(19) **Europäisches Patentamt / European Patent Office / Office européen des brevets**

(11) **EP 4 649 997 A2**

(12) **EUROPEAN PATENT APPLICATION**

(43) Date of publication:
19.11.2025 Bulletin 2025/47

(51) International Patent Classification (IPC):
*A61N 7/02* (2006.01)

(21) Application number: 25205557.9

(22) Date of filing: 18.12.2020

(52) Cooperative Patent Classification (CPC):
A61N 7/02; A61N 2007/0039

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 18.12.2019 US 201962949593 P
18.12.2019 US 201962949595 P

(62) Document number(s) of the earlier application(s) in accordance with Art. 76 EPC:
20845798.6 / 4 076 646

(71) Applicant: Insightec, Ltd.
39120 Tirat Carmel (IL)

(72) Inventors:
• LEVY, Yoav
  Hinanit (IL)
• PRUS, Oleg
  3461407 Haifa (IL)
• AMAR, Talia
  39120 Tirat Carmel (IL)

(74) Representative: Ter Meer Steinmeister & Partner
Patentanwälte mbB
Nymphenburger Straße 4
80335 München (DE)

Remarks:
This application was filed on 30.09.2025 as a divisional application to the application mentioned under INID code 62.

(54) **ADAPTIVE SINGLE-BUBBLE-BASED AUTOFOCUSING AND POWER ADJUSTMENT IN ULTRASOUND PROCEDURES**

(57) Approaches for focusing an ultrasound transducer having multiple transducer elements include generating the first sonication to one or more target regions and measuring the first set of reflection signals resulting from the first sonication; based on the first set of reflection signals, determining whether a target number or a target occurrence rate of focusing events has been reached; if not, generating the second sonication at an adjusted acoustic power to the target region(s) and measuring the second set of reflection signals resulting from the second sonication; and based at least in part on the second set of reflection signals, adjusting a parameter value associated with one or more transducer elements so as to improve an ultrasound focus at the target region(s).

FIG. 6

**Description**

RELATED APPLICATION

This application claims the benefit of and priority to U.S. Provisional Patent Applications Nos. 62/949,593 and 62/949,595 (both filed on December 18, 2019), the entire disclosures of which are hereby incorporated by reference in their entireties.

FIELD OF THE INVENTION

The present invention relates, generally, to systems and methods for ultrasound focusing and, more particularly, to aufocusing using microbubbles and adjusting acoustic power of the ultrasound beam after focusing.

BACKGROUND

Focused ultrasound (i.e., acoustic waves having a frequency greater than about 20 kiloHertz) can be used to image or therapeutically treat internal body tissues within a patient. For example, ultrasound waves may be used in applications involving ablation of tumors, thereby eliminating the need for invasive surgery, targeted drug delivery, control of the blood-brain barrier (BBB), lysing of clots, and other surgical procedures. During tumor ablation, a piezoceramic transducer is placed externally to the patient, but in close proximity to the tissue to be ablated (i.e., the target). The transducer converts an electronic drive signal into mechanical vibrations, resulting in the emission of acoustic waves. The transducer may be geometrically shaped and positioned along with other such transducers so that the ultrasound energy they emit collectively forms a focused beam at a "focal zone" corresponding to (or within) the target tissue region. Alternatively or additionally, a single transducer may be formed of a plurality of individually driven transducer elements whose phases can each be controlled independently. Such a "phased-array" transducer facilitates steering the focal zone to different locations by adjusting the relative phases among the transducers. As used herein, the term "element" means either an individual transducer in an array or an independently drivable portion of a single transducer. Magnetic resonance imaging (MRI) may be used to visualize the patient and target, and thereby to guide the ultrasound beam.

During a focused ultrasound procedure, a series of sonications is applied to cause coagulation necrosis of the target tissue (such as a tumor) without damaging surrounding tissue. If utilized in the central nervous system, the sonications may cause cavitation of microbubbles, which may disrupt blood vessels, thereby inducing "opening" of the BBB for enhancing targeted drug delivery. To achieve these goals, ultrasonic energy emitted from the transducer must be accurately and reliably shaped and focused onto the desired target location. Transducer elements that are not properly configured can lead to improper or suboptimal focal qualities, thereby causing ineffective treatment and/or undesired damage to the non-target tissue. In addition, improperly shaped ultrasound beams may generate unexpected, secondary hot spots at locations other than the intended focal zone; such hot spots may lead to undesired heating, pain for the patient, and/or possibly necrosis of non-targeted tissue.

One source of transducer output errors is inhomogeneity of the intervening tissue (e.g., human skull) through which the ultrasound waves travel prior to reaching the focal zone. The ultrasound waves may interact with the intervening tissue through multiple processes, including propagation, scattering, absorption, reflection, and refraction. For example, tissue inhomogeneity may cause refraction of acoustic energy at the boundaries of regions that have different speeds of sound. Refraction may decrease constructive interference, and hence, the intensity of the acoustic energy at the focal zone. Thus, inhomogeneous tissue may generate beam aberrations and refractions that distort the focus and reduce its intensity, thereby affecting treatment efficiency.

Various approaches have been proposed for calibrating to account for beam aberrations resulting from the intervening tissue. For example, one conventional approach measures phase shifts resulting from travel of an ultrasound beam through the intervening tissue and subsequently adjusts ultrasound parameters to account for the aberrations caused at least in part by the tissue. Typically, this approach uses receiving probes designed for catheter insertion into the brain to measure the amplitude and phase distortion caused by the human skull. Catheter insertions, however, still require surgery, which can be painful and can create a risk of infection.

An alternative, completely noninvasive approach uses X-ray computed tomography (CT) images, rather than receiving probes, to predict the wave distortion caused by the skull. In practice, however, computations of the relative phases alone may too be imprecise to enable high-quality focusing. For example, when ultrasound is focused into the brain to treat a tumor, the skull in the acoustic path may cause aberrations that are not readily ascertainable. In such situations, treatment is typically preceded by a focusing procedure in which an ultrasound focus is generated at or near the target, the quality of the focus is measured (using, e.g., thermal imaging or acoustic radiation force imaging (ARFI)), and experimental feedback is used to adjust the phases of the transducer elements to achieve sufficient focus quality.

The preceding focusing procedure, however, may take a substantial amount of time, which may render it impracticable or, at the least, inconvenient for a patient. In addition, ultrasound energy is inevitably deposited into the tissue surrounding the target during the procedure, thereby potentially damaging healthy tissue. While the effect of pre-therapeutic sonications may be minimized by employing an imaging technique that requires only low acoustic intensity (e.g., ARFI), it is generally desirable to limit the number of sonications prior to treatment.

Another approach to estimating the wave aberrations resulting from the intervening tissue involves use of an acoustic reflector (e.g., a small cloud of microbubbles) in the focus zone. By transmitting the ultrasound waves to the microbubbles and receiving reflections therefrom, the amplitudes and/or phases associated with the reflected ultrasound can be determined; based thereon, aberrations resulting from the intervening tissue can be learned and the transducer parameters (e.g., phase shifts and/or amplitudes) can be adjusted to compensate for the aberrations caused at least in part by the intervening tissue. While this approach may effectively improve the focusing properties at the target, various challenges exists. For example, the microbubble concentration at the target should be in a range that provides significant interaction with the ultrasound waves yet produces reflections signals that are straightforwardly analyzed. In addition, the acoustic power of the ultrasound waves has to be sufficiently large to cause interaction with the microbubbles without, once again, generating undesired artifacts in the reflection signals. Further, because the anatomic properties of the intervening tissue and/or the pharmacokinetics of each patient differ, different microbubble concentrations and different acoustic power may be required for performing the autofocusing procedure on different patients. Accordingly, there is a need for approaches that determine an optimal patient-specific microbubble concentration and acoustic power for performing ultrasound autofocusing in order to achieve a high-quality ultrasound focus at the target.

In some circumstances, the microbubbles may cause undesired damage to the target and/or non-target tissue. For example, the maximal acoustic energy deposited in the focal zone may significantly increase after the transducer parameters are adjusted to improve the focusing properties improve, thereby risking permanent damage to the BBB tissue. When damage to the target/non-target tissue due to energy overexposure is detected during the actual ultrasound procedure, it is, of course, too late to avoid the problem. Accordingly, a need also exists for approaches that create a high-quality ultrasound focus at the target region while avoiding overexposure of the target/non-target region to the acoustic energy.

## SUMMARY

The present invention relates to approaches for automatically focusing ultrasound beams, through an inhomogeneous medium, at a target region utilizing one or more transient reflectors (e.g., one or more microbubbles). The transient reflector(s) may be generated and/or introduced in proximity to (e.g., less than 5 mm away) one or more sonication locations that are in proximity to (e.g., less than 5 mm away) or at the target region. In addition, an optimization approach is implemented to determine one or more optimal values of one or more parameters (e.g., the acoustic power, microbubble concentration, etc.) associated with an ultrasound transducer and/or the acoustic reflector for facilitating the autofocusing procedure. Because optimization can be performed for each patient, the obtained optimal values are patient-specific; as a result, adjustment of the ultrasound parameters (e.g., amplitudes, phases, etc.) using the autofocusing procedure may be more accurate, thereby advantageously improving the focusing properties and treatment efficiency at the target region. It should be understood that the approach of the present invention is useful with any type of transient acoustic reflector, i.e., an acoustic reflector that moves, evolves, and/or dissipates during the period between two measurements. Although the description herein focuses on microbubbles, this is for exemplary purposes only, and references to microbubbles should be understood as interchangeable with other forms of transient acoustic reflector (such as phase-shifting droplets, red blood cells, etc.).

In various embodiments, the optimization approach is commenced by generating and/or introducing an initial concentration of micro bubbles to the sonication location(s) in proximity to or at target region. Preferably, the initial microbubble concentration is sufficiently high to cause significant interaction between the microbubbles and the ultrasound waves but also sufficiently low that the acoustic reflections appear to originate from a point target (e.g., having a size less than that of a quarter of the sonication wavelength) such as a single microbubble (as opposed to a cloud of microbubbles). The initial microbubble concentration may be determined based on, for example, a pre-clinical study, a pre-treatment procedure, and/or from known literature.

Thereafter, at least some of the transducer elements may be activated to transmit a series of low-power (e.g., 7 Watts) sonications to the microbubbles; reflection signals from the microbubbles can be measured. In one embodiment, a signal-selection approach is implemented to select reflection signals from single microbubbles based on consistency among the reflection signals. In one embodiment, a consistency function of the travel times and/or phase delays associated with the reflection signals is defined, and the reflection signals are considered to have sufficient consistency only when the value of the consistency function is maximized or exceeds a predetermined threshold. In various embodiments, the acoustic power of the transmitted sonications is gradually increased until there is a significant (e.g., more than 50% or 100%) change in the parameters (e.g., amplitudes, phases, etc.) associated with the selected reflection signals between two measurements; such reflection signals are referred to as "focusing events." In addition, the acoustic power of the transmitted sonications is continuously increased until a sufficient number (e.g., 10, or in some embodiments, more than 30) of the focusing events is measured. The power for generating the sufficient number of focusing events is referred to as an optimal power and can be utilized during the autofocusing procedure. In some embodiments, an occurrence rate of the focusing events (e.g., the number of focusing events detected in the most recently measured second of time) is calculated, and the power level is adjusted based on the occurrence rate. For example, the power level may be increased when the occurrence rate is too low (e.g., more than 50% or 100% below the target occurrence rate) and decreased when the occurrence rate is too high (e.g., more than 50% or 100% in excess of the target occurrence rate). The target occurrence rate may be one event per second, or in some embodiments, more than 10 events per second.

In some situations, however, prior to a sufficient number (or a sufficient occurrence rate) of the focusing events being detected, the acoustic power of the sonications may have reached a threshold value, where the non-linear responses of the microbubbles (and thereby artifacts in the reflection signals) are detected. Accordingly, in various embodiments, upon determining that the artifacts are present in the reflection signals or that the number of reflection signals having the artifacts is unacceptably high relative to the number of focusing events (e.g., by a factor of two, five or 10), the optimization approach may adjust another parameter such as the microbubble concentration for determining the optimal configurations for facilitating the autofocusing procedure. For example, the microbubble concentration may be increased by 10% or, in some embodiments, 20%; and the applied sonications may then start with the low power again and gradually increase until the optimal power is reached. Again, if the acoustic power reaches the threshold value that causes artifacts in the reflection signals before a sufficient number of focusing events is detected, the microbubble concentration may be further increased. The acoustic power at which a sufficient number of the focusing events are detected indicates the ultrasound power level required to create a therapeutically sufficient acoustic field in the target area. Thus, the acoustic power value corresponding to a sufficient number (or a sufficient occurrence rate) of the focusing events can be used to adjust and/or scale the power level during the ultrasound therapy (or diagnostic) procedure.

Conversely, in other situations, the initial or adjusted microbubble concentration may result in too many focusing events (e.g., larger than 50, or in some embodiments, larger than 80) when the acoustic power is still low (e.g., less than 8 Watts). To save the microbubbles and to reduce the computational complexity of analyzing the reflection signals, in some embodiments, the microbubble concentration is reduced (e.g., by 5%, or in some embodiments, 10%). Again, the sonications may be transmitted at low power, which is gradually increased until the optimal acoustic power is reached. These steps may also be iteratively performed until an optimal microbubble concentration and an optimal acoustic power are obtained. Thereafter, the autofocusing procedure can be commenced using the determined optimal acoustic power and optimal microbubble concentration. Based on the focusing events measured during the autofocusing procedure, the ultrasound parameters (e.g., amplitude and/or phases) can then be adjusted in order to compensate for tissue aberrations, thereby optimizing focusing properties at each sonication location that are in proximity to or at the target region.

Accordingly, various embodiments provide an approach to determining optimal value(s) of one or more parameters (e.g., the acoustic power, microbubble concentration, etc.) associated with the ultrasound transducer and/or the acoustic reflector for performing an accurate and reliable ultrasound autofocusing procedure. Because optimization can be performed for each patient, the obtained optimal values may be patient-specific. As a result, the autofocusing procedure may be used to compensate for aberrations resulting from patient-specific intervening tissue and thereby obtain optimal steering parameters (e.g., ultrasound amplitudes, phases) to focus acoustic energy at the target region.

In addition, various embodiments predict a change in a temperature or a peak power of an ultrasound focus that results from optimization (or at least improvement) of the focusing properties (due to a change in transducer parameters) and, based on the prediction, adjust the transmitted acoustic power level so as to avoid overheating of the target and/or non-target tissue during the ultrasound procedure. In one embodiment, prediction of the peak power/temperature change at each sonication location is based on the phase adjustments determined using reflection signals having sufficient consistency. For example, a physical model and/or an imager (e.g., an MRI apparatus) or other suitable device may be first implemented to predict and/or measure the peak power/temperature change in the first sonication location that results from the amplitude/phase adjustments determined using the sufficiently consistent reflection signals from the reflector(s). Subsequently, based on the measured peak power/temperature change in the first sonication location and comparison of the phase adjustments associated with the reflection signals from the first sonication location against the phase adjustments associated with the reflection signals from other sonication locations (which are determined using the sufficiently consistent reflection signals from the other reflector(s) in the other sonication locations), the peak power/temperature changes in the other sonication locations can be estimated. The discussion herein focuses on temperature change as an exemplary therapeutic and/or acoustic effect, but it should be understood that other such effects may be predicted using the techniques described herein and are within the scope of the invention. Such other effects may include a pressure change, a mechanical index change, a cavitation activity change, a tissue sensitization change, a blood brain barrier disruption change, an acoustic radiation force change and/or a spot shape change.

Additionally or alternatively, the peak power/temperature changes in the other sonication locations may be estimated based on the measured peak power/temperature change in the first sonication location and the phase adjustments associated with the reflection signals from the first sonication and the other sonication locations using the consistency function.

In various embodiments, all (or at least some) transient acoustic reflectors whose reflections are determined to have sufficient consistency may be computationally shifted to coincide at a single location, and the phases associated with the shifted reflection signals and/or the unshifted reflection signal at the coincident location can be determined. In addition, the acoustic power/temperature increase at the coincident location resulting from each of the shifted and/or unshifted reflection signals can be determined (e.g., using the consistency function). Based thereon, the phase associated with the transducer element measuring the reflection signals can be computed as an average or a weighted average of the phases associated with the shifted reflection signals and/or the unshifted reflection signal at the coincident location; and the temperature increase at the coincident location can be estimated as an average or a weighted average of the temperature increases resulting from phase adjustments to the shifted reflection signals and/or the unshifted reflection signal at the coincident location.

In various embodiments, a relationship between one or more ultrasound parameter values (e.g., an acoustic power level, an ultrasound frequency, or an acoustic energy level) and the resulting peak power/temperature change at the target is computationally established using the physical model or empirically determined prior to the ultrasound procedure. Based on the relationship and the predicted peak power/temperature changes in each sonication location, adjustment of the ultrasound parameter value(s) required to compensate for the peak power/temperature change at the target can be determined. Subsequently, the transducer elements can be operated with the adjusted phases, power levels, frequencies, and/or energy levels for generating optimal foci at the sonication locations while avoiding overheating of the target/non-target tissue. In one embodiment, the ultrasound parameter value(s) is adjusted only when the predicted change in the peak power/temperature at the target exceeds a maximum allowable change (e.g., 3°C per second) or when the predicted change causes the peak power/temperature at the target to exceed a target value (e.g., 60°C).

Accordingly, in one aspect, the invention pertains to a system for focusing an ultrasound transducer. In various embodiments, the system includes an ultrasound transducer having multiple transducer elements for providing a series of sonications to one or more target regions; and a controller configured to (a) cause the transducer to generate the first sonication in the sonication series to the target region(s) and measure the first set of reflection signals resulting from the first sonication; (b) based on the first set of reflection signals, determine whether a target number or a target occurrence rate of focusing events has been reached; and (c) if not, (i) cause the transducer to generate the second sonication at an adjusted acoustic power to the target region(s) and measure the second set of reflection signals resulting from the second sonication; and (ii) based at least in part on the second set of reflection signals, adjust a parameter value (e.g., a phase or an amplitude) associated with one or more of the transducer elements so as to improve an ultrasound focus at the target region(s). In one implementation, the focusing events are reflection signals reflected by one or more transient acoustic reflectors located in proximity to or at the target region(s).

The controller may be further configured to determine the focusing events based at least in part on a change in a parameter (e.g., a phase or an amplitude) associated with the first set of the reflection signals. In addition, the controller may be further configured to computationally reconstruct an acoustic field at the target region(s) based at least in part on the first set of reflection signals; and identify the focusing events based at least part on the reconstructed acoustic field. In one embodiment, the focusing events are identified only when the reconstructed acoustic field is converged at the target region(s). In some embodiments, the controller is further configured to select the second set of reflection signals based at least in part on consistency therebetween, and determine the parameter value associated with the transducer element(s) based at least in part on the selected second set of reflection signals. Additionally, the controller may be further configured to, prior to adjusting the parameter value associated with the transducer element(s), repeat (i) determining whether the target number or target occurrence rate of the focusing events has been reached and (ii) if not, causing the transducer to generate the second sonication at the adjusted acoustic power to the target region and measure the second set of reflection signals of the second sonication until the target number or target occurrence rate of the focusing events has been reached. The controller may be further configured to adjust the parameter value associated with the transducer element(s) based at least in part on the adjusted acoustic power.

In various embodiments, the system further includes an administration device for introducing a transient acoustic reflector in proximity to or at the target region(s); the first and/or second set of reflection signals is from the transient acoustic reflector. The administration device may be an automatic administration device or a manual administration device. In addition, the controller may be further configured to determine whether artifacts are present in the first and/or second set of reflection signals; and upon determining that the target number or target occurrence rate of the focusing events has not been reached and the artifacts are presented in the first and/or second sets of the reflection signals, cause the administration device to adjust a parameter (e.g., a concentration, a size and/or an agent type) associated with the transient acoustic reflector. In some embodiments, the controller is further configured to determine whether the artifacts are present in the first set of reflection signals prior to causing the transducer to generate the second sonication. In one embodiment, the controller is further configured to increase the concentration of the acoustic reflector upon determining that the target number or target occurrence rate of the focusing events has not been reached and the artifacts are presented in the first and/or second sets of reflection signals.

The controller may be further configured to, prior to adjusting the parameter value associated with the transducer element(s), determine whether the target number or target occurrence rate of the focusing events has been reached based on the second set of reflection signals; determine whether the adjusted acoustic power associated with the second sonication is below a target power level; and upon determining that the target number or target occurrence rate of the focusing events has been reached and the adjusted acoustic power associated with the second sonication is below the target power level, cause the administration device to decrease a concentration of the transient acoustic reflector. In addition, the controller may be further configured to cause the transducer to generate acoustic energy for creating a transient acoustic reflector in proximity to or at the target region(s); the first and/or second set of reflection signals is from the transient acoustic reflector. The controller may be further configured to cause at least some of the transducer elements to measure the first and/or second set of reflection signals. Additionally or alternatively, the system may further include an acoustic-signal detector for measuring the first and/or second set of reflection signals. In some embodiments, the controller is further configured to (e) predict a therapeutic or acoustic change in the target region(s) resulting from adjustment of the parameter value; (f) if the predicted change exceeds a maximum allowable change or causes the therapeutic or acoustic change in the target region(s) to exceed a target value, determine a power level, an ultrasound frequency and/or an energy level associated with the first one of the transducer elements and/or the second one of the transducer elements for reducing the predicted therapeutic or acoustic change; and (g) activate the ultrasound transducer based at least in part on the parameter value determined in step (d) and the power level, ultrasound frequency or energy level determined in step (f).

In another aspect, the invention relates to a method of focusing an ultrasound transducer having multiple transducer elements. In various embodiments, the method includes (a) generating the first sonication to one or more target regions and measuring the first set of reflection signals resulting from the first sonication; (b) based on the first set of reflection signals, determining whether a target number or a target occurrence rate of focusing events has been reached; and (c) if not, (i) generating the second sonication at an adjusted acoustic power to the target region(s) and measuring the second set of reflection signals resulting from the second sonication; and (ii) based at least in part on the second set of reflection signals, adjusting a parameter value (e.g., a phase or an amplitude) associated with one or more of the transducer elements so as to improve an ultrasound focus at the at least one target region. In one implementation, the focusing events are reflection signals reflected by one or more transient acoustic reflectors located in proximity to or at the target region(s).

The method may further include determining the focusing events based at least in part on a change in a parameter (e.g., a phase or an amplitude) associated with the first set of the reflection signals. In addition, the method may further include computationally reconstructing an acoustic field at the target region(s) based at least in part on the first set of reflection signals; and identifying the focusing events based at least part on the reconstructed acoustic field. In one embodiment, the focusing events are identified only when the reconstructed acoustic field is converged at the target region(s). In some embodiments, the method further includes selecting the second set of reflection signals based at least in part on consistency therebetween, and determining the parameter value associated with the transducer element(s) based at least in part on the selected second set of reflection signals. Additionally, the method may further include, prior to adjusting the parameter value associated with the transducer element(s), repeating (i) determining whether the target number or target occurrence rate of the focusing events has been reached and (ii) if not, causing the transducer to generate the second sonication at the adjusted acoustic power to the target region and measure the second set of reflection signals of the second sonication until the target number or target occurrence rate of the focusing events has been reached. The method may further include adjusting the parameter value associated with the transducer element(s) based at least in part on the adjusted acoustic power.

In various embodiments, the method further includes introducing a transient acoustic reflector in proximity to or at the target region(s); the first and/or second set of reflection signals is from the transient acoustic reflector. In addition, the method may further include determining whether artifacts are present in the first and/or second set of reflection signals; and upon determining that the target number or target occurrence rate of the focusing events has not been reached and the artifacts are presented in the first and/or second sets of the reflection signals, causing the administration device to adjust a parameter (e.g., a concentration, a size and/or an agent type) associated with the transient acoustic reflector. In some embodiments, the artifacts are present in the first set of reflection signals is determined prior to generating the second sonication. In one embodiment, the method further includes increasing the concentration of the acoustic reflector upon determining that the target number or target occurrence rate of the focusing events has not been reached and the artifacts are presented in the first and/or second sets of reflection signals.

The method may further include, prior to adjusting the parameter value associated with the transducer element(s), determining whether the target number or target occurrence rate of the focusing events has been reached based on the second set of reflection signals; determining whether the adjusted acoustic power associated with the second sonication is below a target power level; and upon determining that the target number or target occurrence rate of the focusing events has been reached and the adjusted acoustic power associated with the second sonication is below the target power level, decreasing a concentration of the transient acoustic reflector. In addition, the method may further include generating acoustic energy for creating a transient acoustic reflector in proximity to or at the target region(s); the first and/or second set of reflection signals is from the transient acoustic reflector. The method may further include (e) predicting a therapeutic or acoustic change in the target region(s) resulting from adjustment of the parameter value; (f) if the predicted change exceeds a maximum allowable change or causes the therapeutic or acoustic change in the target region(s) to exceed a target value, determining a power level, an ultrasound frequency and/or an energy level associated with the first one of the transducer elements and/or the second one of the transducer elements for reducing the predicted therapeutic or acoustic change; and (g) activating the ultrasound transducer based at least in part on the parameter value determined in step (d) and the power level, ultrasound frequency and/or energy level determined in step (f).

Another aspect of the invention relates to a system for applying acoustic energy. In various embodiments, the system includes an ultrasound transducer having multiple transducer elements for providing a series of sonications to one or more target regions; and a controller configured to determine a phase value associated with the first one of the transducer elements to be adjusted for generating a focus at the target region(s); predict a therapeutic or acoustic change (e.g., a temperature change, a pressure change, a mechanical index change, a cavitation activity change, a tissue sensitization change, a blood brain barrier disruption change, an acoustic radiation force change and/or a spot shape change) in the target region(s) resulting from adjustment of the phase value; and if the predicted change exceeds a maximum allowable change or causes the therapeutic or acoustic change in the target region(s) to exceed a target value, (i) determine a power level, an ultrasound frequency and/or an energy level associated with the first one of the transducer elements and/or the second one of the transducer elements for reducing the predicted therapeutic or acoustic change, and (ii) activate the ultrasound transducer based at least in part on the determined phase value , power level, ultrasound frequency and/or energy level. In one implementation, the controller is further configured to predict the therapeutic or acoustic change based at least in part on tissue aberrations resulting from intervening tissue located between the ultrasound transducer and the target region(s).

The controller may be further configured to establish a relationship between (i) the change in the target region(s) and (ii) the power level, the ultrasound frequency and/or the energy level associated with the first one of the transducer elements and/or the second one of the transducer elements. The relationship may be established empirically or using a physical model. In addition, the system may further include a transient acoustic reflector located in proximity to or at the target region(s). The controller may be further configured to cause the transducer to generate the first sonication in the sonication series to the target region(s); measure reflection signals of the first sonication from the transient acoustic reflector; select the measured reflection signals based at least in part on consistency therebetween, and based at least in part on the selected reflection signals, determine the phase value associated with the first one of the transducer elements. Alternatively, the controller may be further configured to cause the transducer to generate the first sonication in the sonication series to the target region(s); measure reflection signals of the first sonication from the transient acoustic reflector; computationally reconstruct an acoustic field at the target region(s) based at least in part on the measured reflection signals; select the measured reflection signals based at least in part on the reconstructed acoustic field, and based at least in part on the selected reflection signals, determine the phase value associated with the first one of the transducer elements.

In various embodiments, the system further includes multiple transient acoustic reflectors, each located in proximity to or at one of the target regions; the controller is further configured to sequentially generate multiple sonications to each of the transient acoustic reflectors and measure the reflection signals therefrom; and select the reflection signals associated with the plurality of sonications from the transient acoustic reflectors based at least in part on consistency therebetween. In addition, the controller may be further configured to determine the phase value based at least in part on the first set of the selected reflection signals from the first one of the target regions; and determine a second phase value associated with the first one of the transducer elements based at least in part on a second set of the selected reflection signals from a second one of the target regions. In one embodiment, the controller is further configured to predict the second therapeutic or acoustic change in the second one of the target regions resulting from adjustment of the second phase value based at least in part on the determined phase value, the second phase value and the predicted therapeutic or acoustic change.

In some embodiments, the system further includes a measuring system for measuring the therapeutic or acoustic change in the first one of the target regions due to adjustment of the determined phase value. The controller is further configured to predict the second therapeutic or acoustic change in the second one of the target regions resulting from adjustment of the second phase value based at least in part on the determined phase value, the second phase value and the measured therapeutic or acoustic change in the first one of the target regions. In addition, the controller may be further configured to computationally shift the location of the first one of the transient acoustic reflectors to coincide with the location of the second one of the transient acoustic reflectors; computationally determine an updated phase value associated with the reflection signal from the shifted location of the first one of the transient acoustic reflectors; and predict a therapeutic or acoustic change in the coincident location resulting from adjustments to the updated phase value and the determined phase value based at least in part on the determined phase value, the updated phase value and the predicted therapeutic or acoustic change.

In various embodiments, the system further includes an imaging system, such as a computer tomography (CT) device, a magnetic resonance imaging device (MRI), a positron emission tomography (PET) device, a single-photon emission computed tomography (SPECT) device, or an ultrasonography device, for acquiring images of the target region(s) and/or a non-target region located between the transducer and the target region(s). The controller may be further configured to determine, based at least in part on the acquired images, a spatial configuration of the target region(s) with respect to the transducer and tissue characteristics associated with the target region(s) and the non-target region. In one implementation, the controller is further configured to implement a physical model for (i) predicting the therapeutic or acoustic change in the target region(s) and/or (ii) determining the power level, the ultrasound frequency and/or the energy level based at least in part on the phase value, the spatial configuration of the target region with respect to the transducer and/or the tissue characteristics associated with the target region(s) and the non-target region.

In yet another aspect, the invention pertains to a method of applying acoustic energy from an ultrasound transducer having multiple transducer elements to one or more target regions. In various embodiments, the method includes (a) determining a phase value associated with the first one of the transducer elements to be adjusted for generating an ultrasound focus at the target region(s); (b) predicting a therapeutic or acoustic change (e.g., a temperature change, a pressure change, a mechanical index change, a cavitation activity change, a tissue sensitization change, a blood brain barrier disruption change, an acoustic radiation force change and/or a spot shape change) in the target region(s) resulting from adjustment of the phase value; and (c) if the predicted therapeutic or acoustic change exceeds a maximum allowable change or causes the therapeutic or acoustic change in the target region(s) to exceed a target value, (i) determining a power level, an ultrasound frequency and/or an energy level associated with the first one of the transducer elements and/or the second one of the transducer elements for reducing the predicted therapeutic or acoustic change, and (ii) activating the ultrasound transducer based at least in part on the determined phase value, power level, ultrasound frequency, and/or energy level. In one implementation, the therapeutic or acoustic change is predicted based at least in part on tissue aberrations resulting from intervening tissue located between the ultrasound transducer and the target region(s). In addition, the power level, ultrasound frequency and/or energy level determined in step (c) may reduce the predicted therapeutic or acoustic change to a value not exceeding the target or maximum allowable therapeutic or acoustic change.

The method may further include establishing a relationship between (i) the change in the target region(s) and (ii) the power level, the ultrasound frequency and/or the energy level associated with the first one of the transducer elements and/or the second one of the transducer elements. The relationship may be established empirically or using a physical model. In addition, the method may further include introducing a transient acoustic reflector in proximity to or at the target region(s); generating the first sonication to the target region(s); measuring reflection signals of the first sonication from the transient acoustic reflector; selecting the measured reflection signals based at least in part on consistency therebetween, and based at least in part on the selected reflection signals, determining the phase value associated with the first one of the transducer elements. Alternatively, the method may further include introducing a transient acoustic reflector in proximity to or at the target region(s); generating the first sonication to the target region(s); measuring reflection signals of the first sonication from the transient acoustic reflector; computationally reconstructing an acoustic field at the target region(s) based at least in part on the measured reflection signals; selecting the measured reflection signals based at least in part on the reconstructed acoustic field, and based at least in part on the selected reflection signals, determining the phase value associated with the first one of the transducer elements.

In various embodiments, the method further includes sequentially generating multiple sonications to one or more transient acoustic reflectors located in proximity to or at multiple target regions and measuring the reflection signals therefrom; and selecting the reflection signals associated with the plurality of sonications from the transient acoustic reflectors based at least in part on consistency therebetween. In addition, the method may further include determining the phase value in step (a) based at least in part on the first set of the selected reflection signals from the first one of the target regions; and determining the second phase value associated with the first one of the transducer elements based at least in part on the second set of the selected reflection signals from the second one of the target regions.

In one embodiment, the method further includes predicting the second therapeutic or acoustic change in the second one of the target regions resulting from adjustment of the second phase value based at least in part on the determined phase value, the second phase value and the predicted therapeutic or acoustic change. The method may further include measuring the therapeutic or acoustic change in the first one of the target regions due to adjustment of the determined phase value. In one implementation, the method further includes predicting a second therapeutic or acoustic change in the second one of the target regions resulting from adjustment of the second phase value based at least in part on the determined phase value, the second phase value and the measured therapeutic or acoustic change in the first one of the target regions. In some embodiments, the method further includes computationally shifting the location of the first one of the transient acoustic reflectors to coincide with the location of the second one of the transient acoustic reflectors; computationally determining an updated phase value associated with the reflection signal from the shifted location of the first one of the transient acoustic reflectors; and predicting a therapeutic or acoustic change in the coincident location resulting from adjustments to the updated phase value and the determined phase value based at least in part on the determined phase value, the updated phase value and the predicted therapeutic or acoustic change.

The method may further include acquiring images of the target region(s) and/or a non-target region located between the transducer and the target region(s). Additionally, the method may further include determining, based at least in part on the acquired images, a spatial configuration of the target region(s) with respect to the transducer and tissue characteristics associated with the target region(s) and the non-target region. In one implementation, the method further includes implementing a physical model for (i) predicting the therapeutic or acoustic change in the target region(s) and/or (ii) determining the power level, the ultrasound frequency and/or the energy level based at least in part on the phase value, the spatial configuration of the target region with respect to the transducer and/or the tissue characteristics associated with the target region(s) and the non-target region.

Still another aspect of the invention relates to a system for applying acoustic energy. In various embodiments, the system includes an ultrasound transducer having multiple transducer elements; an imager; and a controller configured to (a) populate a treatment profile specifying multiple treatment parameters associated with the ultrasound transducer and one or more target regions; (b) cause the imager to acquire images of a new target region and/or intervening tissue located between the transducer and the new target region; (c) based at least in part on the acquired images, determine new treatment parameters associated with the new target region; (d) identify best-matching treatment parameters in the treatment profile to the determined new treatment parameters; (e) based at least in part on the identified best-matching treatment parameters, determine (i) adjustment of a phase value associated with one or more of the transducer elements for generating an optimal focus at the new target region and (ii) a therapeutic or acoustic change at the new target region resulting from adjustment of the phase value; and (f) if the determined therapeutic or acoustic change exceeds a maximum allowable change or causes the therapeutic or acoustic change in the target region(s) to exceed a target value, determine adjustment of a power level, a frequency and/or an energy level associated with one or more of the transducer elements for reducing the therapeutic or acoustic change, and activate the ultrasound transducer based at least in part on the adjustment of the phase value determined in step (e) and the determined adjustment of the power level, frequency, and/or energy level.

The treatment parameters may include (i) geometry of the transducer elements and their locations and orientations relative to the target region(s), (ii) tissue characteristics (e.g., a location, a thickness, a density, or a material property) of the target region(s) and the intervening tissue, (iii) adjustments of the phase values associated with at least some of the transducer elements for generating an optimal focus at the target region(s), (iv) therapeutic or acoustic changes at the target region(s) resulting from the phase value adjustments, and/or (v) adjustments of the acoustic powers, frequencies and/or energy levels associated with at least some of the transducer elements for reducing the therapeutic or acoustic changes. In addition, the controller may be further configured to empirically determine the adjustments of the phase values in step (iii), the therapeutic or acoustic changes at the target region(s) in step (iv), and the adjustments of the acoustic powers, frequencies and/or energy levels in step (v). In one embodiment, the controller is further configured to implement a physical model for determining the adjustments of the phase values in step (iii), the therapeutic or acoustic changes at the target region(s) in step (iv), and the adjustments of the acoustic powers, frequencies and/or energy levels in step (v) based at least in part on the geometry of the transducer elements and their locations and orientations relative to the target region(s) and the tissue characteristics of the target region(s) and the intervening tissue.

In another aspect, the invention relates to a method of applying acoustic energy utilizing an ultrasound transducer having multiple transducer elements. In various embodiments, the method includes (a) populating a treatment profile specifying multiple treatment parameters associated with the ultrasound transducer and one or more target regions; (b) acquiring images of a new target region and/or intervening tissue located between the transducer and the new target region; (c) based at least in part on the acquired images, determining new treatment parameters associated with the new target region; (d) identifying best-matching treatment parameters in the treatment profile to the determined new treatment parameters; (e) based at least in part on the identified best-matching treatment parameters, determining (i) adjustment of a phase value associated with one or more of the transducer elements for generating an optimal focus at the new target region and (ii) a therapeutic or acoustic change at the new target region resulting from adjustment of the phase value; and (f) if the determined therapeutic or acoustic change exceeds a maximum allowable change or causes the therapeutic or acoustic change in the target region(s) to exceed a target value, (i) determining adjustment of a power level, a frequency and/or an energy level associated with one or more of the transducer elements for reducing the therapeutic or acoustic change, and (ii) activating the ultrasound transducer based at least in part on the adjustment of the phase value determined in step (e) and the determined adjustment of the power level, frequency, and/or energy level.

The treatment parameters may include (i) geometry of the transducer elements and their locations and orientations relative to the target region(s), (ii) tissue characteristics (e.g., a location, a thickness, a density, or a material property) of the target region(s) and the intervening tissue, (iii) adjustments of the phase values associated with at least some of the transducer elements for generating an optimal focus at the target region(s), (iv) therapeutic or acoustic changes at the target region(s) resulting from the phase value adjustments, and/or (v) adjustments of the acoustic powers, frequencies and/or energy levels associated with at least some of the transducer elements for reducing the therapeutic or acoustic changes. In addition, the method may further include empirically determining the adjustments of the phase values in step (iii), the therapeutic or acoustic changes at the target region(s) in step (iv), and the adjustments of the acoustic powers, frequencies and/or energy levels in step (v). In one embodiment, the method further includes implementing a physical model to determine the adjustments of the phase values in step (iii), the therapeutic or acoustic changes at the target region(s) in step (iv), and the adjustments of the acoustic powers, frequencies and/or energy levels in step (v) based at least in part on the geometry of the transducer elements and their locations and orientations relative to the target region(s) and the tissue characteristics of the target region(s) and the intervening tissue.

In still another aspect, the invention relates to a system for focusing an ultrasound transducer. In various embodiments, the system includes an ultrasound transducer having multiple transducer elements for providing a series of sonications to one or more target regions; and a controller configured to (a) cause the transducer to generate the first sonication in the sonication series to the target region(s) and measure the first set of reflection signals resulting from the first sonication; (b) based on the first set of reflection signals, determine whether a target number or a target occurrence rate of focusing events has been reached; (c) if not, cause the transducer to generate the second sonication at an adjusted acoustic power to the target region(s) and measure the second set of reflection signals resulting from the second sonication; (d) based at least in part on the second set of reflection signals, adjust a phase value associated with one or more of the transducer elements for improving an ultrasound focus at the target region(s); (e) predict a therapeutic or acoustic change in the target region(s) resulting from adjustment of the parameter value; and (f) if the predicted change exceeds a maximum allowable change or causes the therapeutic or acoustic change in the target region(s) to exceed a target value, (i) determine a power level, an ultrasound frequency and/or an energy level associated with the first one of the transducer elements and/or the second one of the transducer elements for reducing the predicted therapeutic or acoustic change, and (ii) activate the ultrasound transducer based at least in part on the determined parameter value, power level, ultrasound frequency and/or energy level.

In another aspect, the invention pertains to a method of focusing an ultrasound transducer having multiple transducer elements. In various embodiments, the method includes (a) generating the first sonication to one or more target regions and measuring the first set of reflection signals resulting from the first sonication; (b) based on the first set of reflection signals, determining whether a target number or a target occurrence rate of focusing events has been reached; (c) if not, generating the second sonication at an adjusted acoustic power to the target region(s) and measuring the second set of reflection signals resulting from the second sonication; (d) based at least in part on the second set of reflection signals, adjusting a parameter value associated with one or more of the transducer elements so as to improve an ultrasound focus at the target region(s); (e) predicting a therapeutic or acoustic change in the target region(s) resulting from adjustment of the parameter value; and (f) if the predicted change exceeds a maximum allowable change or causes the therapeutic or acoustic change in the target region(s) to exceed a target value, (i) determining a power level, an ultrasound frequency and/or an energy level associated with the first one of the transducer elements and/or the second one of the transducer elements for reducing the predicted therapeutic or acoustic change, and (ii) activating the ultrasound transducer based at least in part on the determined parameter value, power level, ultrasound frequency and/or energy level.

As used herein, the term "substantially" means $\pm 10\%$, and in some embodiments, $\pm 5\%$. Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, steps, or characteristics may be combined in any suitable manner in one or more examples of the technology. The headings provided herein are for convenience only and are not intended to limit or interpret the scope or meaning of the claimed technology.

BRIEF DESCRIPTION OF THE DRAWINGS

In the drawings, like reference characters generally refer to the same parts throughout the different views. Also, the drawings are not necessarily to scale, with an emphasis instead generally being placed upon illustrating the principles of the invention. In the following description, various embodiments of the present invention are described with reference to the following drawings, in which:

FIG. 1 schematically depicts an exemplary ultrasound system in accordance with various embodiments;
FIG. 2A depicts one or more transient acoustic reflectors located in proximity to one or more target regions in accordance with various embodiments;
FIG. 2B depicts application of sonications to multiple locations in proximity to a target region in accordance with various embodiments;
FIG. 3A depicts reflection signals measured from the transient reflector(s) and differential signals determined based on the measured reflection signals in accordance with various embodiments;

FIG. 3B schematically depicts maximal amplitudes associated with differential signals of the measured reflection signals in accordance with various embodiments;

FIGS. 4A and 4B illustrate a confined and an unconfined acoustic field, respectively, computationally reconstructed at a sonication location in accordance with various embodiments;

FIG. 5 schematically depicts a relationship between an acoustic power of the applied sonications and a change in the reflection signals in accordance with various embodiments;

FIG. 6 is a flow chart illustrating an approach for automatically focusing ultrasound beams at a target region in accordance with various embodiments;

FIG. 7A schematically illustrates reflection signals measured from transient reflectors in accordance with various embodiments;

FIG. 7B schematically depicts computationally shifting the location of one transient acoustic reflector to coincide with the location of another transient acoustic reflector in accordance with various embodiments;

FIG. 8 schematically depicts a relationship between an acoustic power level of the applied sonications and the resulting temperature change at the target region in accordance with various embodiments; and

FIGS. 9A-9C are flow charts illustrating approaches for predicting a change in the temperature or peak power of an ultrasound focus resulting from optimization/improvement of the focusing properties and, based thereon, adjusting one or more ultrasound parameters so as to avoid overheating of the target and/or non-target tissue in accordance with various embodiments.

## DETAILED DESCRIPTION

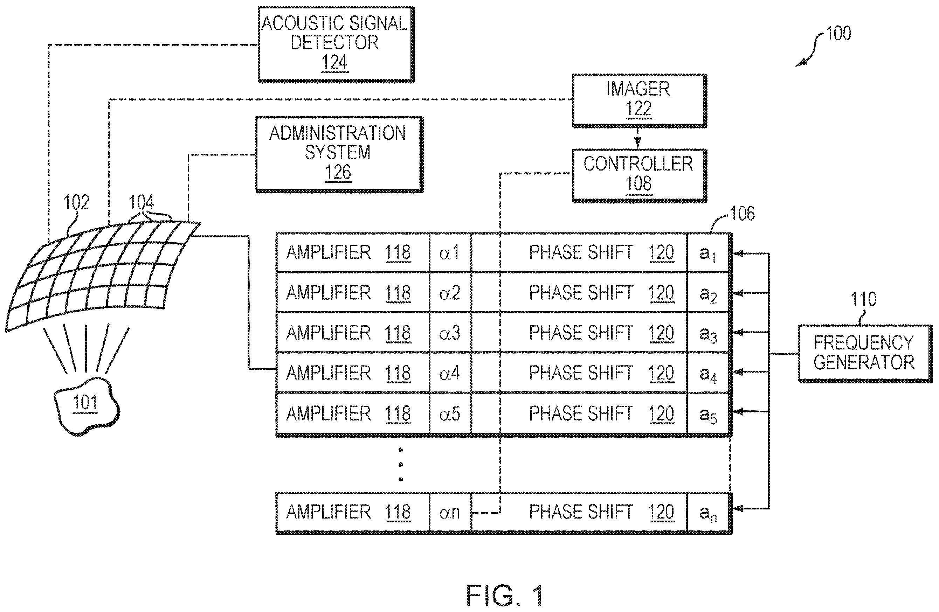

FIG. 1 illustrates an exemplary ultrasound system 100 for focusing ultrasound beams through the skull onto a target region 101 within a patient's brain. One of ordinary skill in the art, however, will understand that the ultrasound system 100 described herein may be applied to any part of the human body. In various embodiments, the system 100 includes a phased array 102 of transducer elements 104, a beamformer 106 driving the phased array 102, a controller 108 in communication with the beamformer 106, and a frequency generator 110 providing an input electronic signal to the beamformer 106.

The array 102 may have a curved (e.g., spherical or parabolic) shape suitable for placement on the surface of the skull or a body part other than the skull, or may include one or more planar or otherwise shaped sections. Its dimensions may vary, depending on the application, between millimeters and tens of centimeters. The transducer elements 104 of the array 102 may be piezoelectric ceramic elements, and may be mounted in silicone rubber or any other material suitable for damping the mechanical coupling between the elements 104. Piezo-composite materials, or generally any materials capable of converting electrical energy to acoustic energy, may also be used. To assure maximum power transfer to the transducer elements 104, the elements 104 may be configured for electrical resonance at 50 $\Omega$, matching input connector impedance.

The transducer array 102 is coupled to the beamformer 106, which drives the individual transducer elements 104 so that they collectively produce a focused ultrasonic beam or field. For n transducer elements, the beamformer 106 may contain n driver circuits, each circuit including or consisting of an amplifier 118 and a phase delay circuit 120; each drive circuit drives one of the transducer elements 104. The beamformer 106 receives a radio frequency (RF) input signal, typically in the range from 0.1 MHz to 1.0 MHz, from the frequency generator 110, which may, for example, be a Model DS345 generator available from Stanford Research Systems. The input signal may be split into n channels for the n amplifiers 118 and delay circuits 120 of the beamformer 106. In some embodiments, the frequency generator 110 is integrated with the beamformer 106. The radio frequency generator 110 and the beamformer 106 are configured to drive the individual transducer elements 104 of the transducer array 102 at the same frequency, but at different phases and/or different amplitudes.

The amplification or attenuation factors $\alpha_1$-$\alpha_n$ and the phase shifts $a_1$-$a_n$ imposed by the beamformer 106 serve to transmit and focus ultrasonic energy through inhomogeneous tissue (e.g., the patient's skull) onto the target region (e.g., a region in the patient's brain). Via adjustments of the amplification factors and/or the phase shifts, a desired shape and intensity of a focal zone may be created at the target region.

The amplification factors and phase shifts may be computed using the controller 108, which may provide the computational functions through software, hardware, firmware, hardwiring, or any combination thereof. For example, the controller 108 may utilize a general-purpose or special-purpose digital data processor programmed with software in a conventional manner, and without undue experimentation, to determine the frequency, phase shifts and/or amplification factors of the transducer elements 104. In certain embodiments, the controller computation is based on information about the characteristics (e.g., structure, thickness, density, etc.) of the skull and their effects on propagation of acoustic energy. In various embodiments, such information is obtained from an imager 122, such as a magnetic resonance imaging (MRI) device, a computer tomography (CT) device, a positron emission tomography (PET) device, a single-photon emission computed tomography (SPECT) device, or an ultrasonography device. The imager 122 may provide a set of two-dimensional images suitable for reconstructing a three-dimensional image of the skull from which thicknesses and densities can be inferred; alternatively, image acquisition may be three-dimensional. In addition, image-manipulation functionality may be implemented in the imager 122, in the controller 108, or in a separate device.

The system 100 may be modified in various ways within the scope of the invention. For example, the system may further include an acoustic-signal detector (e.g., a hydrophone) 124 that measures transmitted or reflected ultrasound, and which may provide the signals it receives to the controller 108 for further processing. The reflection and transmission signals may also provide an alternative or additional source for determining the phase shifts and/or amplification factors or feedback for the phase and amplitude adjustments of the beamformer 106 as further described below. The system 100 may contain a positioner for arranging the array 102 of transducer elements 104 with respect to the patient's skull. In order to apply ultrasound therapy to body parts other than the brain, the transducer array 102 may take a different (e.g., cylindrical) shape. In some embodiments, the transducer elements 104 are mounted movably and rotatably, providing mechanical degrees of freedom that can be exploited to improve focusing properties. Such movable transducers may be adjusted by conventional actuators, which may be driven by a component of controller 108 or by a separate mechanical controller.

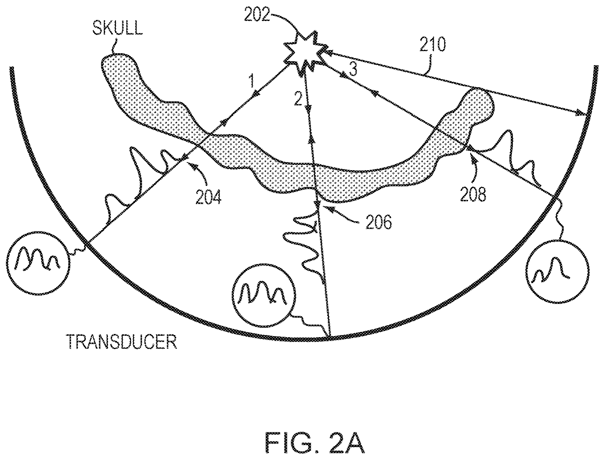

Referring to FIG. 2A, in various embodiments, one or more transient reflectors 202 are introduced in proximity to (e.g., less than 5 mm away) one or more sonication locations that are in proximity to or at the target region 101 for facilitating autofocusing of the ultrasound waves. The microbubbles 202 may be generated by applying acoustic energy from the transducer elements 104 to the target 101. The microbubbles 202 can be formed due to the negative pressure produced by the propagating ultrasonic waves or when the heated liquid ruptures and is filled with gas/vapor. Because of their encapsulation of gas, the microbubble(s) 202 may act as reflectors of the ultrasound waves and transmit coherent omnidirectional signals 204-208 to the transducer 102; the reflection signals 204-208 can be substantially concurrently detected by the transducer elements 104 and/or acoustic signal detector 124 associated therewith as further described below. Based on analysis of the reflection signals, the controller 108 may obtain information of the focusing properties at the target region 101 and subsequently adjust the transducer configurations (e.g., phase shifts and/or amplitudes) so as to compensate for the aberrations caused by the intervening tissue 210 located between the transducer elements 104 and target 101, thereby optimizing (or at least improving) the focusing properties at the target region. Approaches to generating microbubbles utilizing ultrasound waves are provided, for example, in U.S. Patent Publication No. 2019/0308038, the entire content of which is incorporated herein by reference; and approaches to utilizing the microbubbles to optimize/improve focusing properties of the acoustic beams are provided, for example, in PCT Publication No. WO 2020/128615, the entire contents of which are incorporated herein by reference.

Additionally or alternatively, the acoustic reflector 202 may be introduced into the patient's body intravenously; the transient reflector may either be injected systemically into the patient or locally into the target region 101 using an administration system 126. For example, the transient reflector 202 may include or consist of one or more microbubbles introduced into the patient's brain in the form of liquid droplets that subsequently vaporize to form the microbubbles; or as gas-filled bubbles entrained within a liquid carrier, e.g., a conventional ultrasound contrast agent. Alternatively, other substances suitable for cavitation nucleation can be administered instead of bubbles (see, e.g., https://www.springer.com/cda/content/document/cda_downloaddocument/9783642153426-c1.pdf?SGWID=0-0-45-998046-p174031757).

Typically, an automated administration system may be operated by the controller 108 or a dedicated controller associated with the administration system. For example, analysis of the reflection signals may cause the controller 108 to operate the administration device 126 so as to increase or decrease the amount of acoustic reflectors introduced intravenously and/or adjust the type of microbubbles. Alternatively or additionally, analysis of the reflection signals may cause the controller 108 to operate the transducer 102 so as to increase or decrease, via induced cavitation, the amount of circulating or localized microbubbles; an increased acoustic power, for example, may reduce the number of microbubbles by causing their collapse. Alternatively, the administration system 126 may be manual, such as a simple syringe. In the case of manual administration, analysis of the reflection signals may cause the controller 108 to determine an adjustment to the amount of acoustic reflectors introduced intravenously and/or the type of microbubbles, and to operate the transducer 102 based on this determination. Approaches for providing acoustic reflectors to the target region using suitable administration systems are described, for example, in PCT Publication No. WO 2019/116095, the entire disclosure of which is hereby incorporated by reference.

In one embodiment, the administration system 126 introduces a relatively low concentration (e.g., 5% of the concentration used for standard imaging) of microbubbles into the target 101 such that the acoustic reflections appear to originate from a point target (e.g., having a size less than that of a quarter of the sonication wavelength) such as a single microbubble (as opposed to a cloud of microbubbles). This is because reflection signals from a cloud of microbubbles may be incoherent and/or exhibit artifacts due to low SNRs and/or vibrations from the multiple microbubbles; as a result, analysis of the reflection signals from the cloud of microbubbles may be inaccurate and adjustment of the transducer parameters based thereon may be insufficient to account for the aberrations caused by the intervening tissue. In addition, analysis of the reflection signals from the cloud of microbubbles may be computationally expensive and time-consuming. On the other hand, the microbubble concentration is preferred to be sufficiently high to cause significant interaction with the ultrasound waves, thereby providing detectable reflections for performing the autofocusing procedure. Thus, in one embodiment, prior to the autofocusing procedure, an initial microbubble concentration introduced to the target region is first empirically predetermined based on a pre-clinical study, a pre-treatment procedure, and/or from known literature. Thereafter, an optimization approach is performed to determine the optimal microbubble concentration, acoustic power and/or other parameters associated with the ultrasound transducer and/or microbubbles for facilitating the autofocusing procedure as further described below.

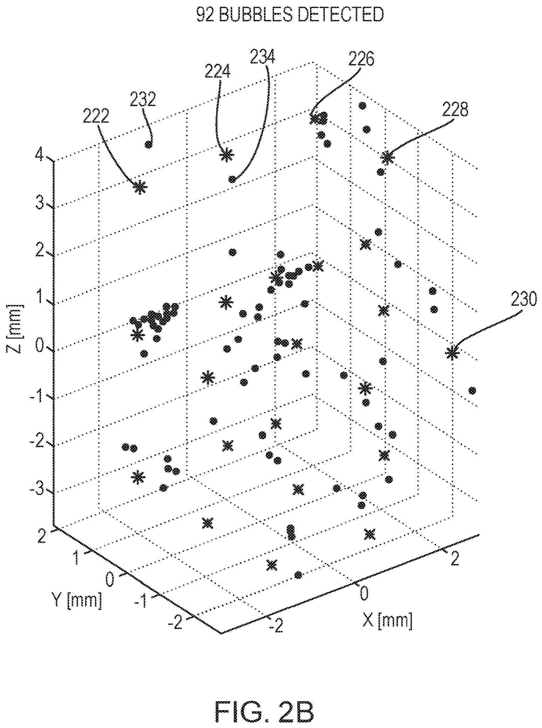

In an exemplary case, the initial microbubble concentration contains 1.3 milliliter (mL) of a microbubble suspension (acquired from, for example, DEFINITY) diluted in 500 mL of water; the infusion is then introduced into the patient's body with a drip rate of 1 mL/minute. Referring to FIG. 2B, after introduction of the microbubbles 202, the controller 108 may activate at least some of the transducer elements 104 to sequentially generate multiple foci at various sonication locations 222-230 that are in proximity to the target region 101 (e.g., less than 5 mm away) or at the target region 101, and each location may have one or more transient reflectors 202 associated therewith. For example, the transducer elements 104 may generate one or more series of sonications to the first sonication location 222 and measure the reflections from the transient reflector 232 located in proximity thereto. Subsequently, the transducer elements 104 may generate another one or more series of sonications to the second sonication location 224 and measure the reflections from the transient reflector 234 associated therewith. This process may continue until a desired number of reflection signals (e.g., at least 10) from all (or at least some) of the sonication locations in proximity to the target 101 are measured.

In various embodiments, the sonication locations 222-230 are determined based on the image(s) acquired by the imager 122 and/or the ultrasound transducer 102. For example, the imager 122 may acquire images of the target and/or non-target regions; and the ultrasound transducer 102 may acquire images of the transient reflector(s) 202 in the target/non-target regions based on the reflection signals therefrom. Based on the acquired images of the target/non-target regions and the transient reflectors associated therewith, the controller 108 may select the sonication locations 222-230 that are near (e.g., less than 5 mm away) and/or at the target region and having one or more transient reflectors in proximity to thereto (e.g., less than 5 mm away). Approaches to acquiring images of the transient reflector(s) using the reflection signals therefrom are provided, for example, in a U.S. Patent Application No. 62/949,597 (filed on December 18, 2019), the entire disclosure of which is hereby incorporated by reference.

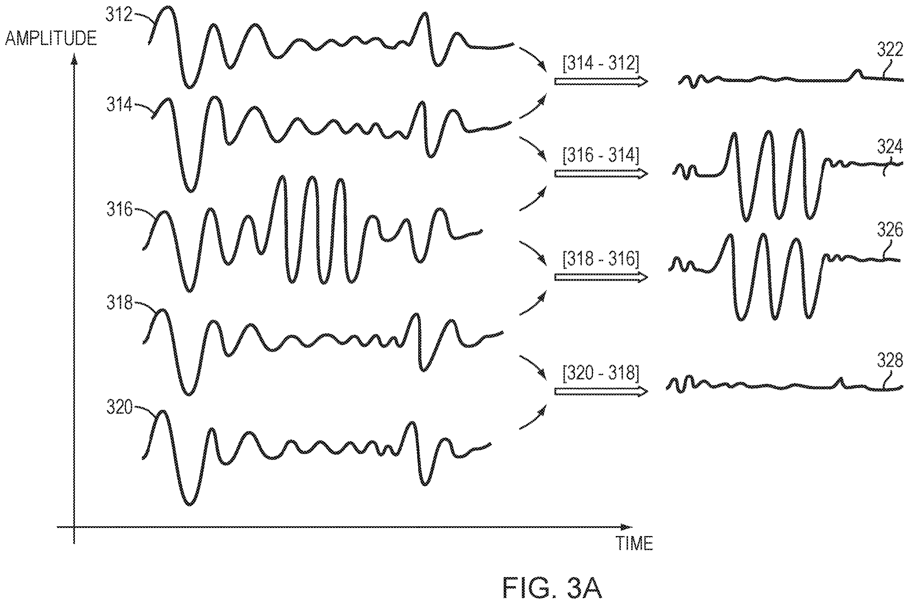

In various embodiments, the sonications are first transmitted at a low acoustic power (e.g., in the range of 2 to 50 Watts); reflections from the tissue and microbubbles are then measured and analyzed as further described below. Typically, the reflection signals resulting from the low-power transmission may not differ significantly from each other. Accordingly, in one embodiment, the acoustic power of the transmitted sonications is gradually increased until there is a significant change in the parameters (e.g., amplitudes, phases, etc.) associated with the reflection signals between two consecutive measurements. For example, FIG. 3A illustrates five reflection signals 312-320 from the first sonication location 222 measured by a transducer element E. Upon receiving the reflection signals, the controller 108 may implement an initial signal-processing approach to obtain reflection signals from the transient acoustic reflector 232 (as opposed to the background reflectors). In one embodiment, the controller 108 first computes a differential signal 322 representing the difference between the reflection signals 314, 312 (obtained, e.g., by subtracting the signal 312 from the signal 314), a differential signal 324 representing the difference between the reflection signals 314, 316, a differential signal 326 representing the difference between the reflection signals 316, 318, and a differential signal 328 representing the difference between the reflection signals 318, 320. In this example, signals 312, 314, 318 and 320 are essentially background signals and signal 316 is a combination of reflection from transient signals and background signals. Therefore, the differential signals 324 and 326 are approximately clean reflection signals from the transient reflector 232. It should be understood that reflection signals may be analyzed as they are received, i.e., without waiting for subsequent signals or sets of signals.

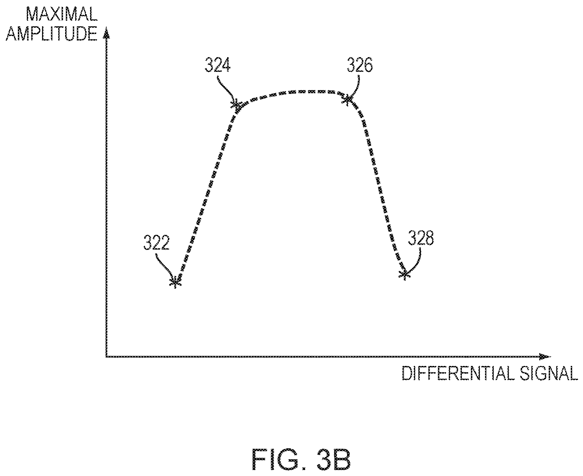

Referring to FIG. 3B, subsequently, the controller 108 may compare the values of the maximal amplitudes associated with the differential signals 322, 324, 326, 328. As depicted, because there is a significant change in the amplitudes (e.g., by more than 50% or 100%) between the signals 322, 324, the signal 324 can be selected to determine the focusing properties at the first sonication location 222. As used herein, the signal 324 is referred to as a "focusing event," and the acoustic power of the transmitted waves that starts to generate the focusing event is referred to as the first threshold power, $P_1$.

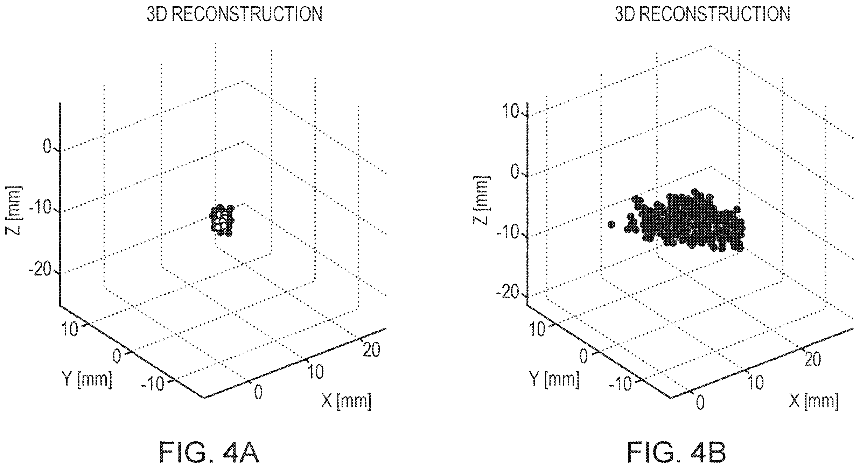

In some embodiments, the focusing event is identified or selected based on the acoustic field at the sonication location. For example, referring again to FIG. 2B, the controller may computationally reconstruct the acoustic field at sonication location 222 based on the measured reflection signals from the transient reflector 232 in proximity thereto. Referring to FIG. 4A, in one embodiment, when the reconstructed acoustic field is confined (e.g., spanning a volume of less than 10 mm$^3$ or, in some embodiments, 20 mm$^3$), the reflection signal that the confined acoustic field is reconstructed based on is identified as the focusing event. Referring to FIG. 4B, when the reconstructed acoustic field is unconfined (e.g., spanning a volume of less than 50 mm$^3$ or, in some embodiments, 80 mm$^3$), the measured signal may be discarded. Alternatively or additionally, the controller 108 may computationally reconstruct the acoustic field based on information of the tissue aberrations at the target/non-target region. Approaches for computationally reconstructing the acoustic field based on tissue aberrations are provided, for example, in U.S. Patent Application No. 62/949,597 (filed on December 18, 2019) and PCT Patent Application entitled "Systems and Methods for Providing Tissue Information in an Anatomic Target Region Using Acoustic Reflectors" filed on even date herewith, the entire contents of which are incorporated herein by reference.

Identification and selection of the focusing events may advantageously allow the reflection signals from the transient reflectors (e.g., microbubbles), as opposed to the background reflectors, to be selected for further analysis. This is because typically, reflection signals from the background reflectors are relatively invariant between two consecutive measurements, whereas reflection signals from the transient reflectors 202 may exhibit a relatively significant change between two consecutive measurements as the transient reflector moves, evolves or dissipates during the measurement interval. Thus, the differential signals with relatively small amplitude changes likely originate with the background reflectors; in contrast, reflection signals that have relatively large amplitude changes may be more likely from the transient reflectors. As used herein, the term "transient reflector" refers to an acoustic reflector that dissipates or evolves with time during sonications (e.g., microbubbles) and the term "background reflector" refers to an acoustic reflector that does not dissipate or evolve significantly during sonication (e.g., the skull).

In addition, a signal-selection approach may be implemented to select the reflection signals that are from single microbubbles. In one embodiment, the signal-selection approach selects the reflection signals based on the consistency therebetween. For example, the reflection signals are considered to have sufficient consistency when the value of a consistency function of the phase delays (or travel times) associated with the reflection signals is maximized or exceeds a predetermined threshold - e.g., 40%). By using the reflection signals having sufficient consistency, artifacts exhibited in the signals due to low SNRs and/or vibrations from the multiple microbubbles may advantageously be eliminated (or at least reduced), this thereby provides more accurate information about the focusing properties at the target region. In addition, the computational complexity of analyzing the reflection signals can be significantly reduced. Further details regarding signal selection are provided, for example, in PCT Publication No. WO 2020/128615, the entire contents of which are incorporated herein by reference.

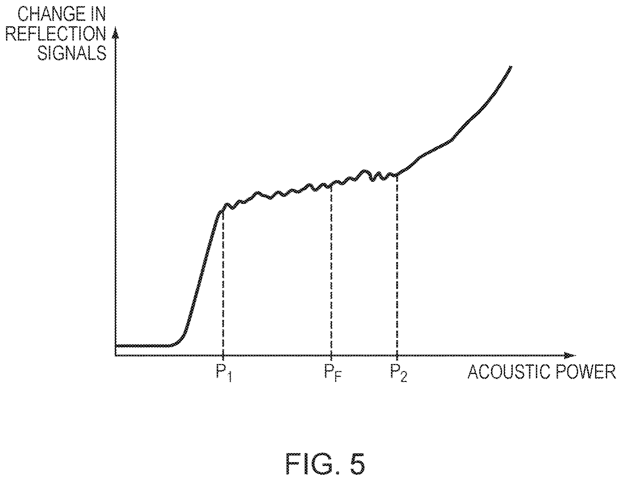

Generally, the larger the acoustic power of the applied sonications, the larger the number of generated focusing events at the target region will be. Thus, referring to FIG. 5, in various embodiments, the optimization approach commences by gradually increasing the acoustic power of sonications to cause more focusing events until a sufficient number (e.g., 10, or in some embodiments, more than 30) or a sufficient occurrence rate (e.g., one event per second, or in some embodiments, more than 10 events per second) of the focusing events is measured for determining the focusing properties at the sonication location(s) with a desired degree of accuracy and reliability; up to a point (e.g., before non-linear responses of the microbubbles occur), more measurements produce greater accuracy. The power for generating the sufficient number (or sufficient occurrence rate) of focusing events is referred to as an optimal power, $P_F$, and can be utilized during the autofocusing procedure - i.e., the ultrasound waves are transmitted with the determined power level, $P_F$, thereby causing sufficient interaction with the microbubbles at the sonication locations that are in proximity to or at the target region. In some situations, however, prior to a sufficient number or a sufficient occurrence rate of the focusing events being detected, the acoustic power may have reached the second threshold value, $P_2$, where the non-linear responses of the microbubbles (and thereby artifacts in the reflection signals) are detected. Artifacts may significantly reduce the consistency between the reflection signals and thereby cause inaccurate determination of the focusing properties at the target region. Accordingly, upon determining that artifacts are present in the reflection signals or that the number of reflection signals having artifacts exceeds the number of focusing events (i.e., the non-consistent reflection signals exceed the sufficiently consistent reflection signals in number), the optimization approach may adjust another parameter, different from the acoustic power of the transmitted waves, to improve autofocusing performance.

For example, the microbubble concentration introduced to the patient's body may be adjusted (e.g., increasing it by 10% or, in some embodiments, by 20%). The applied sonications may then start with a low power (e.g., 7 Watts) again, and the power may be gradually increased until the optimal power $P_F$ is reached. Again, if the acoustic power reaches the second threshold value $P_2$ before a sufficient number of focusing events is detected, the microbubble concentration may be further increased. These processes may be alternately and iteratively performed until a sufficient number of the focusing events is detected before the acoustic power reaches the second threshold $P_2$.

Conversely, in other situations, the initial or adjusted microbubble concentration may result in too many focusing events (e.g., larger than 50, or in some embodiments, larger than 80) when the acoustic power is still low (e.g., less than 8 Watts); this indicates that too many microbubbles are present at the sonication location(s), increasing the computational complexity of analyzing the reflection signals and/or wasting the microbubbles. Accordingly, the microbubble concentration may be reduced (e.g., by 5%, or in some embodiments, 10%) and the sonications may, again, be transmitted at low power (e.g., 7 Watts), with power gradually increased until the optimal acoustic power $P_F$ is reached for performing autofocusing. These processes may also be alternately and iteratively performed until an optimal concentration of the microbubbles is introduced for providing a sufficient amount (or a sufficient occurrence rate) of detectable focusing events at a sufficiently large (e.g., 10 Watts) acoustic power $P_F$.

It should be noted that the above-described optimization approach for determining optimal acoustic power and/or microbubble concentration to facilitate the ultrasound focusing procedure is exemplary only; any suitable approach to optimizing the values of any parameters associated with the ultrasound wave and microbubbles for performing the autofocusing procedure may be used and all such approaches are thus within the scope of the invention. For example, the optimization approach may adjust the transmission pattern of the sonications, the agent type and/or the size of the microbubbles in order to generate a sufficient amount (or a sufficient occurrence rate) of focusing events for determining the focusing properties at the target region.

After the optimal acoustic power and/or the optimal microbubble concentration are determined, in various embodiments, the ultrasound autofocusing procedure can be commenced by introducing the microbubbles at the determined concentration and activating the transducer 102 to transmit sonications having the determined optimal acoustic power, $P_F$, to the microbubbles at the sonication location(s) in proximity to or at the target region. Reflections from the microbubbles can then be measured using, for example, the acoustic-signal detector 124. Additionally or alternatively, the transducer elements 104 may possess both transmit and detect capabilities. Thus, at least some of the transducer elements 104 may be configured to measure acoustic signals reflected from the target region. Approaches to configuring the transducer elements 104 for detecting reflected signals are provided, for example, in PCT Publication No. WO 2019/234497, the contents of which are incorporated herein by reference.

The measured signals may be provided to the controller 108 to obtain information, such as the amplitudes and/or phases, associated with the reflections; these may be compared to the amplitudes and/or phases associated with the transmitted ultrasound waves from the transducer elements 104. Based on the deviations therebetween, the controller 108 may adjust the transducer parameters (e.g., phase shifts and/or amplitudes) so as to compensate for the aberrations caused by the intervening tissue 206 located between the transducer elements 104 and target 101, thereby improving the focusing properties at the target. In one embodiment, adjustments of the ultrasound parameters to compensate for the tissue aberrations are determined based only on focusing events (e.g., reflection signals having sufficient consistency or corresponding to a confined acoustic field at the target) so as to reduce computational complexity and increase measurement accuracy of the focusing properties at the target region. Approaches for autofocusing an ultrasound beam at the target region are provided, for example, in PCT Publication Nos. WO 2018/020315 and WO 2020/128615; the entire contents of these applications are incorporated herein by reference.

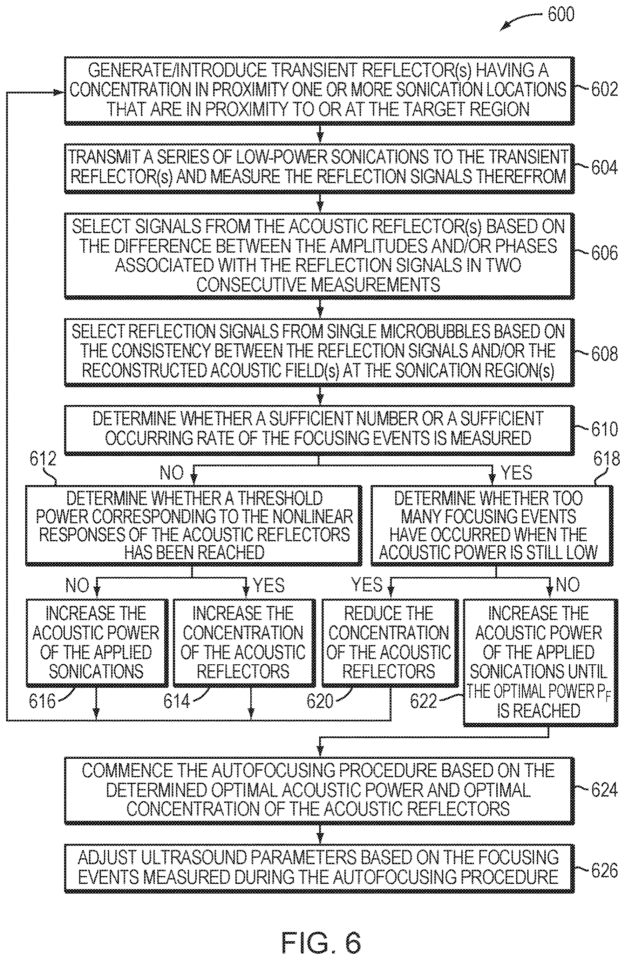

FIG. 6 illustrates an exemplary approach 600 for automatically focusing ultrasound beams, through an inhomogeneous medium, at a target region utilizing one or more transient reflectors (e.g., one or more microbubbles). In a first step 602, one or more transient reflectors (e.g., microbubbles) having an initial concentration are generated and/or introduced in proximity to (e.g., less than 5 mm away) one or more sonication locations that are in proximity to or at the target region. The initial microbubble concentration can be determined based on, for example, a pre-clinical study, a pre-treatment procedure, and/or from known literature. In a second step 604, at least some of the transducer elements 104 are activated to transmit a series of low-power (e.g., 7 Watts) sonications to the transient reflector(s), and reflection signals from the tissue and/or transient reflector(s) are measured using, for example, the acoustic-signal detector 124 and/or the transducer elements 104. In a third step 606, the controller 108 may implement an initial signal-processing approach to obtain reflection signals from the transient acoustic reflector(s) (as opposed to the background reflector(s)). In one embodiment, reflection signals from the transient acoustic reflector(s) are determined based on the difference between the amplitudes and/or phases associated with the reflection signals in two consecutive measurements. Additionally or alternatively, a signal-selection approach may be implemented to select reflection signals from single microbubbles based on the consistency between the reflection signals (step 608). For example, a consistency function of the travel times and/or phase delays associated with the reflection signals is defined, and the reflection signals are considered to have sufficient consistency only when the value of the consistency function is maximized or exceeds a predetermined threshold (e.g., 40%). Subsequently, the controller 108 may, based on the selected reflection signals, determine whether a sufficient number (e.g., 10, or in some embodiments, more than 30) or a sufficient occurrence rate (one event per second, or in some embodiments, more than 10 events per second) of the focusing events is detected (step 610). In one embodiment, the focusing event is identified based on a comparison of the parameter values (e.g., amplitudes, phases, etc.) associated with the selected reflection signals between two measurements. The focusing event occurs only when there is a significant (e.g., more than 50% or 100%) change in the parameter values. Alternatively, the focusing event may be identified based on a computationally reconstructed acoustic field at the sonication location. The acoustic field reconstruction may be based on the measured reflection signals from the transient reflector(s) and/or the information of tissue aberrations at the target/non-target region. In some embodiments, when the controller 108 determines that a sufficient number or sufficient occurrence rate of the focusing events has not yet been reached, the controller determines whether a threshold power corresponding to the non-linear responses of the acoustic reflectors has been reached (step 612). For example, the non-linear responses may be determined based on the artifacts present in the reflection signals or the ratio of the number of reflection signals having artifacts to the number of focusing events. If the acoustic power of the sonications has reached the threshold value, the microbubble concentration may be increased (e.g., by 5%, or in some embodiments, 10%) (step 614). Otherwise, the power of the applied sonications may be increased (step 616). In various embodiments, when a sufficient number (or a sufficient occurrence rate) of the focusing events is measured, the controller 108 determines whether too many focusing events (e.g., larger than 50, or in some embodiments, larger than 80) have occurred (or too high of an event occurrence rate is detected) when the acoustic power is still low (e.g., less than 8 Watts) (step 618). If so, the concentration of the acoustic reflectors may be reduced (e.g., by 5%, or in some embodiments, 10%) (step 620). Otherwise, the power of the applied sonications may be gradually increased until the optimal power $P_F$ is reached (step 622). Steps 602-622 may be iteratively performed until the optimal concentration of the microbubbles and the optimal acoustic power are obtained. Thereafter, the autofocusing procedure can be commenced using the determined optimal acoustic power and optimal concentration of the acoustic reflectors (step 624). In addition, based on the focusing events measured during the autofocusing procedure, the ultrasound parameters (e.g., amplitude and/or phases) can be adjusted in order to compensate for tissue aberrations, thereby optimizing focusing properties at each sonication location that are in proximity to or at the target region (step 626).

Accordingly, various embodiments provide an approach for determining optimal value(s) of one or more parameters (e.g., the acoustic power, microbubble concentration, etc.) associated with the ultrasound transducer and/or the acoustic reflector 202 for performing an accurate and reliable ultrasound autofocusing procedure. Because optimization can be performed for each patient, the obtained optimal values may be patient-specific. As a result, the autofocusing procedure may be used to compensate for aberrations resulting from patient-specific intervening tissue and thereby obtain optimal steering parameters (e.g., ultrasound amplitudes, phases) to focus acoustic energy at the target region.

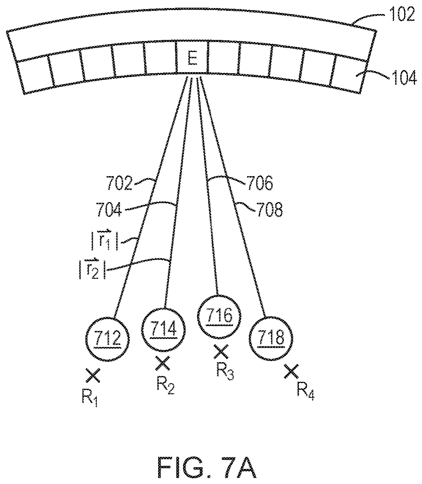

In various embodiments, after an optimal focus is created at the target region, the peak power or deposited acoustic energy at the target increases, thereby causing undesired damage to the target and/or non-target tissue. Thus, it may be desired to estimate the peak power/energy increase at the target due to improvement of the focusing properties and adjust an ultrasound parameter (e.g., the acoustic power level applied by the transducer elements) to compensate for the peak power/energy increase. In various embodiments, estimation of the peak power/energy increase at the target 101 is based on the sufficiently consistent reflection signals received from various sonication locations that are in proximity to (e.g., less than 5 mm away) or at the target 101. For example, FIG. 7A illustrates four sufficiently consistent reflection signals 702-708 received from the transient reflectors 712-718 located in proximity to (e.g., less than 5 mm away) sonication locations $R_1$ - $R_4$, respectively, which are in proximity to or at the target 101. Typically, the sonication locations are sufficiently proximate spatially (e.g., 2 mm apart) that differences in aberrations resulting from, for example, the movements and/or changes in the intervening tissue located between the transducer elements and the sonication locations $R_1$ - $R_4$ can be ignored. In one embodiment, a physical model and/or the imager 122 (or other suitable device) is implemented to predict and/or measure the temperature increase $\Delta T_1$ at the sonication location $R_1$ that results from the phase adjustment, $\varphi_1$, associated with the transducer element $E$ for generating an optimal focus at the first sonication location $R_1$. The controller 108 may then compare, for example, the phase adjustment, $\varphi_2$, for generating an optimal focus at the second sonication location $R_2$ against $\varphi_1$, and based on the comparison and the predicted/measured temperature increase $\Delta T_1$ at the sonication location $R_1$, estimate the temperature increase $\Delta T_2$ at the sonication location $R_2$. Similar approaches can be used to estimate the temperature increases $\Delta T_3$ and $\Delta T_4$ at the sonication locations $R_3$ and $R_4$, respectively.

In some embodiments, the physical model includes a consistency function $f(\vec{r'})$:

$$f\left(\vec{r'}\right) = \left| \frac{\sum_{\text{all elements}} W \times e^{-i\left(\varphi_1 - \varphi_2 + \omega\left(\frac{dr}{c}\right)\right)}}{\sum_{\text{all elements}} W} \right| \qquad \text{Eq. (1),}$$

where $W$ denotes a weighting factor; c denotes the average sound velocity in the target area; $\omega = 2\pi f$, where $f$ represents the frequency of the reflection signals 702, 704; $r' = \vec{r_1} - \vec{r_2}$, where $\vec{r_i}$ is the geometric location of the i$^{th}$ transient reflector; and $dr = |\vec{r_1}| - |\vec{r_2}|$ for each transducer element, where $|\vec{r_1}|$ and $|\vec{r_2}|$ denote the distances between the transducer element $E$ and the transient reflectors 712, 714, respectively. Because the temperature increases $\Delta T_1$ and $\Delta T_2$ highly correlates to the phase adjustments $\varphi_1$ and $\varphi_2$ associated with the sufficiently consistent reflection signals 702, 704, respectively, the temperature increase $\Delta T_2$ may be estimated using the consistency function $f(\vec{r'})$ and $\Delta T_1$.

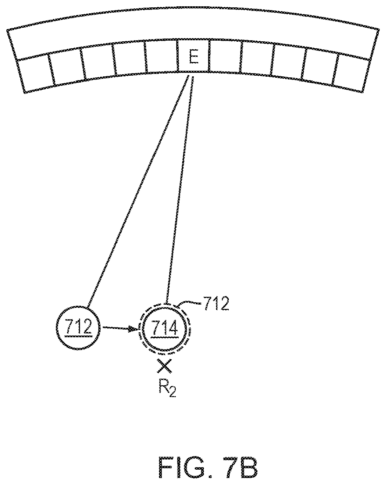

Referring to FIG. 7B, in various embodiments, the controller 108 computationally shifts the location of one of the two transient acoustic reflectors (e.g., reflector 712) associated with the two consistent reflection signals to coincide with the location of the other transient acoustic reflector (e.g., reflector 714). In addition, the controller 108 can computationally determine the phase adjustment, $\varphi'_1$, associated with the reflection signal 702 from the shifted location of the reflector 712. Further, as described above, the physical model and/or imager 122 may predict and/or measure the temperature increase $\Delta T_2$ at the sonication location $R_2$ resulting from adjustment of the phase $\varphi_2$ associated with the reflection signal 204. Again, by comparing the phase adjustments $\varphi'_1$ and $\varphi_2$ associated with the shifted reflection signal 702 and unshifted reflection signal 704, respectively, and/or based on the predicted/measured temperature increase $\Delta T_2$ at the

sonication location $R_2$ and the consistency function, the controller 108 may estimate the temperature increase $\Delta T_1$' at the sonication location $R_2$ resulting from adjustment of the phase $\varphi_1'$ associated with the shifted reflection signal 702. In some embodiments, the temperature increase $\Delta T_1$' at the sonication location $R_2$ is determined by comparing the phase adjustments $\varphi_1'$ and $\varphi_1$ associated with the shifted and unshifted reflection signal 702, respectively, the predicted/-measured temperature increase $\Delta T_1$ and/or the consistency function. Similar approaches can be used to estimate the temperature increases $\Delta T_3$' and $\Delta T_4$' at the sonication locations $R_2$ resulting from shifting the locations of the acoustic reflectors 716, 718, respectively, associated with the reflection signals 706, 708 to coincide with the location of the acoustic reflector 714. In various embodiments, the phase adjustment associated with the transducer element $E$ for generating an optimal focus at the coincident location (e.g., the location associated with the reflector 714) can then be computed as an average or a weighted average of the phases associated with the shifted reflection signals 702, 706, 708 and/or the unshifted reflection signal 704 at the coincident location. In addition, the temperature increase at the sonication location $R_2$ can be estimated as an average or a weighted average of the temperature increases $\Delta T_1$', $\Delta T_2$, $\Delta T_3$' and $\Delta T_4$'.

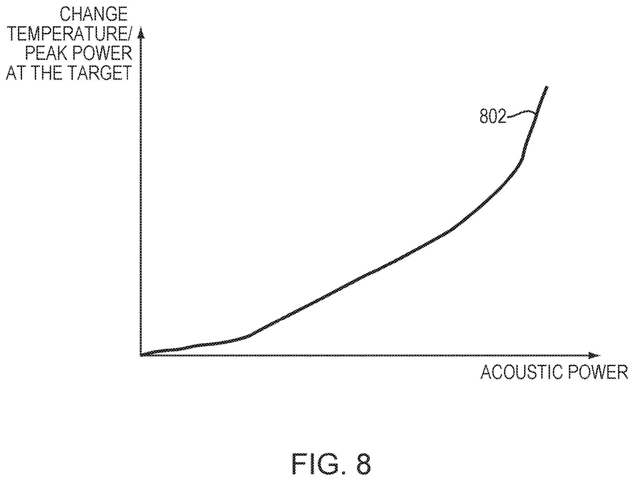

Referring to FIG. 8, in some embodiments, a relationship 802 between an ultrasound parameter (e.g., the acoustic power level applied by the transducer elements 104, the ultrasound frequency and/or the acoustic energy level) and the resulting temperature/peak power change at the target (and/or the sonication location(s)) can be established and stored in a database prior to the ultrasound procedure. The relationship may be empirically obtained from a pre-clinical study, a pre-treatment procedure, and/or from known literature. For example, the relationship may be modeled empirically based on measurements performed using an *ex-vivo* skull. Alternatively, the relationship may be computationally determined using the physical model. For example, using conventional techniques implemented without undue experimentation, the physical model may predict the focusing properties (e.g., the shape, size, location, and peak acoustic power of the focus zone) based on information about the geometry of the transducer elements 104 and their locations and orientations relative to the target region 101, as well as the power levels and phases of ultrasound waves transmitted from the elements 104. In addition, the physical model may include parameters, such as material properties (e.g., the energy absorption of the tissue or the speed of sound at the employed frequency) of the target tissue and intervening tissue along the beam path for predicting aberrations resulting from the intervening tissue and/or the temperature at the target 101 upon application of the ultrasound waves. The material properties may be collected using the imager 122 as described above and/or other suitable devices. For example, based on the acquired images, a tissue model characterizing the material characteristics of the intervening tissue and the target tissue may be established. The tissue model may take the form of a 3D table of cells corresponding to the voxels representing the intervening and/or target tissue; the cells have attributes whose values represent characteristics of the tissue, such as the absorption coefficient, that are relevant to the energy absorption. The voxels are obtained tomographically by the imager 122 and the type of tissue that each voxel represents can be determined automatically by conventional tissue-analysis software. Using the determined tissue types and a lookup table of tissue parameters (e.g., absorption coefficient by type of tissue), the cells of the tissue model may be populated. Further detail regarding creation of a tissue model that identifies the energy absorption coefficient, heat sensitivity and/or thermal energy tolerance of various tissues may be found in U.S. Patent Publication No. 2012/0029396, the entire disclosure of which is hereby incorporated by reference.

In various embodiments, based on the relationship between the ultrasound parameter(s) (e.g., the frequency, acoustic power level applied by the transducer elements, and/or acoustic energy level) and the resulting temperature change at the target (and/or the sonication location(s)) and the estimated temperature increases $\Delta T_{2-4}$, at the sonication locations $R_{2-4}$, respectively, the controller 108 may determine adjustment of the ultrasound parameter to compensate for the estimated temperature increases $\Delta T_{2-4}$. Based thereon, the transducer elements may then be activated with the adjusted ultrasound parameter(s) (e.g., power level, frequency and/or energy level) and adjusted phases for generating high-quality foci at the sonication locations $R_{2-4}$, respectively, while avoiding overheating of the target/non-target tissue.

In various embodiments, the relationship between the changes in the phases associated with the transducer elements and the peak power/temperature changes at the focus can be computationally estimated using the physical model as well. For example, by providing certain inputs, such as the expected phase adjustments associated with the transducer elements, the physical model together with the tissue model can be used to compute the peak power/-temperature increase at the target 101. This approach may obviate the need to measure the temperature increase $\Delta T_1$ at the sonication location $R_1$, thereby advantageously avoiding overheating of the tissue at the sonication location $R_1$.

Alternatively, the relationship between the phase adjustments associated with the transducer elements and the peak power/temperature change at the focus, as well as the relationship between the peak power/temperature change at the focus and the ultrasound parameter(s) (e.g., the frequency, acoustic power level applied by the transducer elements, and/or acoustic energy level), may be computationally determined using the physical model and tissue model and/or empirically determined based on a look-up treatment profile of patients who previously experienced the ultrasound procedure. The treatment profile may be stored in a database in memory accessible by the controller 108 and may specify treatment parameters, such as the geometry of the transducer elements 104 and their locations and orientations relative to the target region 101, the tissue characteristics (e.g., locations, thickness, densities, material properties, etc.) of the target tissue and intervening tissue, the required phase adjustments for generating an optimal focus at the target region, the peak power/temperature increase at the focus resulting from the phase adjustments, adjustments of the acoustic power, frequency and/or energy level associated with the transducer elements to compensate for the peak power/temperature increase so as to avoid overheating of the target/non-target tissue, etc. In various embodiments, prior to performing the ultrasound procedure on the current patient, the imager 122 is activated to acquire images associated with the target 101 of the current patient, and based thereon, it is straightforward to determine the tissue characteristics associated with the target tissue and intervening tissue and the geometry of the location and orientation of the target 101 relative to the transducer elements. The controller 108 may then look up the treatment profile that most closely matches the current treatment parameters using one or more conventional matching algorithms (e.g., block-matching algorithms, phase-correlation and frequency-domain methods, pixel recursive algorithms, Bayesian, optical flow methods, etc.). Based thereon, the controller 108 may determine the required phase adjustments for generating an optimal focus at the target 101 as well as the required power level/energy level/frequency adjustments associated with the transducer elements to compensate for the temperature increase resulting from improvement of the focusing properties at the focus (due to adjustments of the phases). Again, the transducer elements can subsequently be activated based on the determined phase and power adjustments.

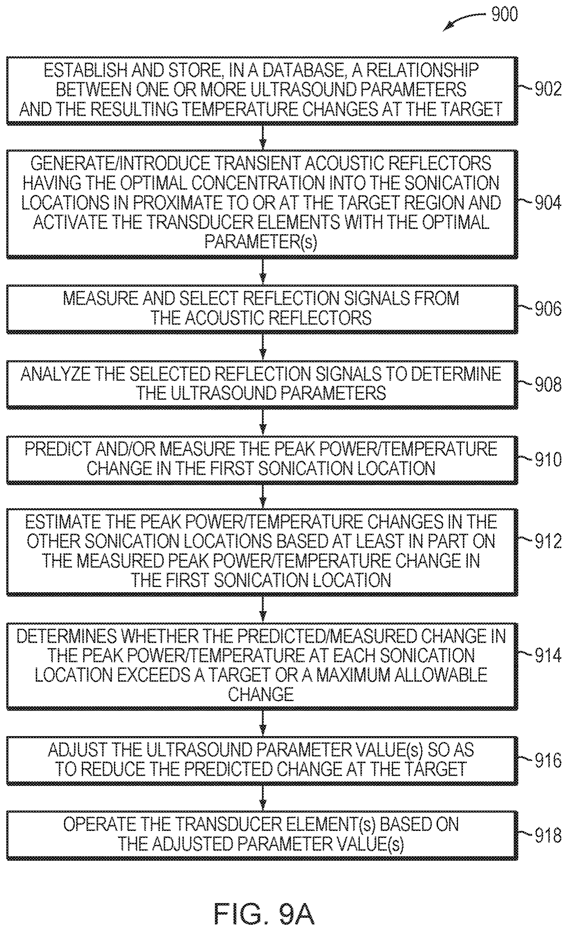

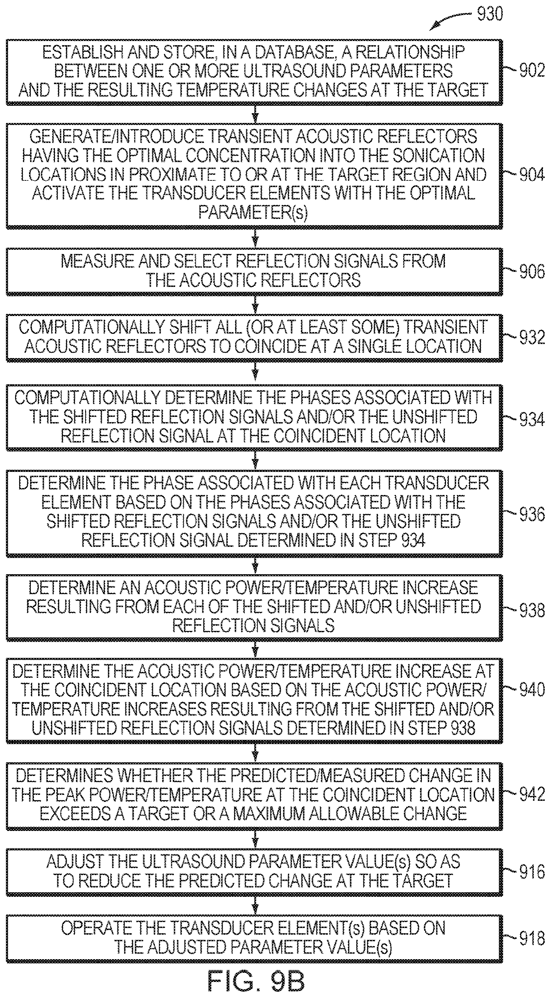

FIGS. 9A and 9B illustrate exemplary approaches 900, 930 for predicting/measuring a change in the temperature or peak power of an ultrasound focus resulting from optimization (or at least improvement) of the focusing properties and, based thereon, adjusting one or more ultrasound parameters (e.g., the acoustic power level, ultrasound frequency and/or acoustic energy level) so as to avoid overheating of the target and/or non-target tissue during the ultrasound procedure. In a first step 902, a relationship between one or more ultrasound parameters and the resulting temperature changes at the target (and/or the sonication locations) is established and stored in a database prior to the ultrasound procedure. The relationship may be computationally determined using a physical model and/or empirically obtained from a pre-clinical study, a pre-treatment procedure, and/or from known literature. The ultrasound procedure may be commenced by generating/introducing transient acoustic reflectors (e.g., microbubbles) having the optimal concentration (e.g., determined using the approach described in FIG. 6) into the sonication locations in proximity to or at the target region and activating the transducer elements with the optimal ultrasound parameter(s), such as the acoustic power level determined in approach 600, to treat the target (step 904). Reflection signals from the acoustic reflectors may then be measured and selected (e.g., based on the consistency therebetween) (step 906). In addition, the selected reflection signals may be analyzed to determine ultrasound parameters (e.g., amplitude and/or phases) of the transducer elements to compensate for aberrations resulting from the intervening tissue, thereby optimizing (or at least improving) focusing properties at each sonication location that are in proximity to or at the target region (step 908).

In one embodiment, a physical model and/or an imager is implemented to predict and/or measure the peak power/temperature change in the first sonication location (step 910). Based on the predicted/measured peak power/-temperature change in the first sonication location and comparison of the phase adjustments associated with the reflection signals from the first sonication location against the phase adjustments associated with the reflection signals from other sonication locations, the peak power/temperature changes in the other sonication locations can be estimated (step 912). In one embodiment, the controller 108 determines whether the predicted/measured change in the peak power/temperature at each sonication location exceeds a maximum allowable change (e.g., 3°C per second) or causes the peak power/temperature at the target to exceed a target value (e.g., 60°C) (step 914); and if so, the controller may, based on the predicted/measured peak power/temperature change in each sonication location and the relationship established in step 902, adjust the ultrasound parameter value(s) (e.g., power level, frequency and/or energy level) so as to reduce the predicted change at the sonication location (step 916). Subsequently, the transducer element(s) may be operated based on the adjusted parameter value(s) (step 918).

Referring to FIG. 9B, in some embodiments, all (or at least some) transient acoustic reflectors whose reflections are determined to have sufficient consistency may be computationally shifted to coincide at a single location (step 932), and the phases associated with the shifted reflection signals and/or the unshifted reflection signal at the coincident location can be computationally determined (step 934). The phase associated with each transducer element measuring the reflection signals can then be computed as an average or a weighted average of the phases associated with the shifted reflection signals and/or the unshifted reflection signal at the coincident location (step 936). In addition, the acoustic power/temperature increase at the coincident location resulting from each of the shifted and/or unshifted reflection signals can be determined (e.g., using the consistency function) (step 938). The peak power/temperature increase at the coincident location can then be estimated as an average or a weighted average of the temperature increases resulting from the phase adjustments of the shifted reflection signals and/or the unshifted reflection signal at the coincident location (step 940). In some embodiments, the controller 108 then determines whether the estimated peak power/temperature change in the coincident location exceeds the maximum allowable change or causes the peak power/temperature at the target to exceed the target value (step 942). If so, the controller may, based on the predicted/measured peak power/temperature change in the coincident location and the relationship established in step 902, adjust the ultrasound parameter value(s) of one or more transducer elements so as to compensate for the peak power/temperature change (step 916), thereby avoiding overheating of the target and/or non-target tissue. Thereafter, the transducer elements can be operated based on the adjusted parameter value(s) (step 918).

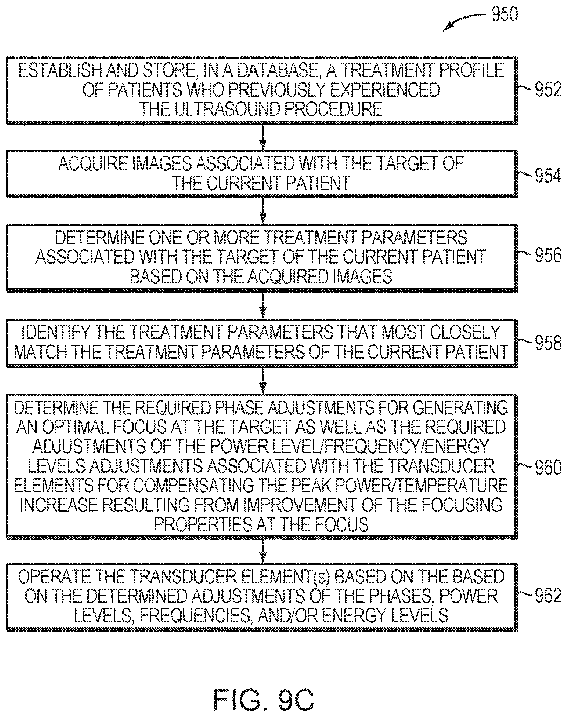

FIG. 9C illustrates another approach 950 for predicting/measuring a change in the temperature or peak power of an ultrasound focus resulting from optimization (or at least improvement) of the focusing properties and, based thereon, adjusting one or more ultrasound parameters (e.g., the acoustic power level, ultrasound frequency and/or acoustic energy level) so as to avoid overheating of the target and/or non-target tissue during the ultrasound procedure. In a first step 902, prior to the ultrasound procedure, a treatment profile of patients who previously experienced the ultrasound procedure is established and stored in a database accessible to the controller 108. The treatment profile may specify various treatment parameters, such as the geometry of the transducer elements and their locations and orientations relative to the target region, the tissue characteristics of the target tissue and/or intervening tissue, the required phase adjustments for generating an optimal focus at the target region, the peak power/temperature increase at the focus resulting from the phase adjustments, the acoustic power adjustments associated with the transducer elements to compensate for the peak power/temperature increase so as to avoid overheating the target/non-target tissue, etc. In addition, the treatment profile may include (i) a relationship between the ultrasound parameters (e.g., the acoustic power levels, ultrasound frequencies and/or acoustic energy levels) and the resulting temperature changes at the target (and/or the sonication locations) and (ii) a relationship between the phase adjustments associated with the transducer elements 104 and the peak power/-temperature changes at the focus. In one embodiment, prior to performing the ultrasound procedure on the current patient, the imager 122 is activated to acquire images associated with the target 101 of the current patient (step 954). Based on the images, one or more treatment parameters (e.g., tissue characteristics associated with the target tissue and/or intervening tissue and the geometry of the location and orientation of the target relative to the transducer elements) associated with the current patient can be determined (step 956). The controller 108 may then look up the treatment profile to identify the treatment parameters that most closely match the treatment parameters of the current patient (step 958), and based thereon determine the required phase adjustments for generating an optimal focus at the target 101 as well as the required power level/frequency/energy level adjustments to compensate for the peak power/temperature increase resulting from optimization/improvement of the focusing properties at the focus (due to adjustments of the phases) (step 960). Subsequently, the transducer elements can be operated based on the determined adjustments of the phases, power levels, frequencies, and/or energy levels (step 962).

In some embodiments, the ultrasound parameter adjustment approaches 900, 930, 950 described above are implemented when properties of the intervening tissue change during the ultrasound procedure. For example, during a long sonication procedure for ablating a brain tumor, the properties of the skull through which the ultrasound waves travel before reaching the target tumor may change due to the heat accumulated therein. To compensate for the change in the properties of the skull and maintain the focusing properties at the target, the configuration (e.g., amplitudes and/or phases) associated with the transducer elements may have to be adjusted (because, again, after the phase adjustments, the energy deposited at the focal zone may significantly increase and can cause adverse effects at the target/non-target tissue). Accordingly, the acoustic-power adjustment approach described above may be implemented to reduce the acoustic power levels applied by the transducer elements 104, thereby compensating for the peak power/energy increase at the focal zone.

As used herein, the terms "optimal" and "optimizing" generally involve a substantial improvement (e.g., by more than 10%, more than 20%, or more than 30%) over the prior art, but do not necessarily imply achievement of the best theoretically possible energy absorption. In addition, functionality for performing autofocusing of ultrasound beams and adjusting the ultrasound parameters (e.g., phases, acoustic power levels, frequencies, acoustic energy levels, etc.), such as selecting reflection signals from the acoustic reflectors based on the difference between the amplitudes and/or phases associated with the reflection signals in two consecutive measurements, computationally reconstructing acoustic fields at the sonication locations, selecting reflection signals from single acoustic reflectors based on the consistency between the reflection signals and/or the reconstructed acoustic fields, determining whether a sufficient number (or a sufficient occurrence rate) of the focusing events is detected, determining whether a threshold power corresponding to the non-linear responses of the acoustic reflectors has been reached, causing the acoustic power of the applied sonications to be increased or reduced, causing the concentration of the acoustic reflectors to be increased or reduced, determining whether too many focusing events have occurred when the acoustic power is still low, commencing the autofocusing procedure based on the determined optimal acoustic power and optimal concentration of the acoustic reflectors, adjusting the ultrasound parameters based on the focusing events measured during the autofocusing procedure, establishing a relationship between the ultrasound parameters and the resulting temperature changes at the target either empirically or using a physical model, predicting the peak power/temperature change in the first sonication location, estimating the peak power/temperature changes in the other sonication locations based at least in part on the predicted/measured peak power/temperature change in the first sonication location, determining whether the predicted/measured change in the peak power/temperature at each sonication location exceeds a maximum allowable change or causes the peak power/-temperature at the target to exceed a target value, adjusting the ultrasound parameter value(s) so as to reduce the predicted peak power/temperature change at the sonication locations, computationally shifting all (or at least some) transient acoustic reflectors to coincide at a single location, computationally determining the phases associated with the shifted reflection signals and/or the unshifted reflection signal at the coincident location, determining the phase associated with each transducer element based on the phases associated with the shifted reflection signals and/or the unshifted reflection signal, determining the acoustic power/temperature increase resulting from each of the shifted and/or unshifted reflection signals, determining the acoustic power/temperature increase at the coincident location based on the acoustic power/temperature increases resulting from the shifted and/or unshifted reflection signals, establishing a treatment profile of patients who previously experienced the ultrasound procedure, acquiring images associated with the target region of the current patient, determining one or more treatment parameters associated with the target of the current patient based on the acquired images, identifying the treatment parameters in the treatment profile that most closely match the treatment parameters of the current patient, and determining the required phase adjustments for generating an optimal focus at the target of the current patient as well as the required adjustments of the power level/frequency/energy level associated with the transducer for reducing the acoustic power/temperature change, as described above in FIGS. 6 and 9A-9C, whether integrated within a controller of the imager, the administration system, and/or an ultrasound system, or provided by a separate external controller, may be structured in one or more modules implemented in hardware, software, or a combination of both. For embodiments in which the functions are provided as one or more software programs, the programs may be written in any of a number of high level languages such as PYTHON, FORTRAN, PASCAL, JAVA, C, C++, C#, BASIC, various scripting languages, and/or HTML. Additionally, the software can be implemented in an assembly language directed to the microprocessor resident on a target computer (e.g., the controller); for example, the software may be implemented in Intel 80x86 assembly language if it is configured to run on an IBM PC or PC clone. The software may be embodied on an article of manufacture including, but not limited to, a floppy disk, a jump drive, a hard disk, an optical disk, a magnetic tape, a PROM, an EPROM, EEPROM, field-programmable gate array, or CD-ROM. Embodiments using hardware circuitry may be implemented using, for example, one or more FPGA, CPLD or ASIC processors.

In addition, the term "controller" used herein broadly includes all necessary hardware components and/or software modules utilized to perform any functionality as described above; the controller may include multiple hardware components and/or software modules and the functionality can be spread among different components and/or modules.

Certain embodiments of the present invention are described above. It is, however, expressly noted that the present invention is not limited to those embodiments; rather, additions and modifications to what is expressly described herein are also included within the scope of the invention.

It follows a list of examples:

1. A system for focusing an ultrasound transducer, the system comprising:

an ultrasound transducer comprising a plurality of transducer elements for providing a series of sonications to at least one target region; and
a controller configured to:

(a) cause the transducer to generate a first sonication in the sonication series to the at least one target region and measure a first set of reflection signals resulting from the first sonication;
(b) based on the first set of reflection signals, determine whether a target number or a target occurrence rate of focusing events has been reached; and
(c) if not,

(i) cause the transducer to generate a second sonication at an adjusted acoustic power to the at least one target region and measure a second set of reflection signals resulting from the second sonication; and
(ii) based at least in part on the second set of reflection signals, adjust a parameter value associated with at least one of the transducer elements so as to improve an ultrasound focus at the at least one target region.

2. The system of example 1, wherein the focusing events are reflection signals reflected by at least one transient acoustic reflector located in proximity to or at the at least one target region.

3. The system of example 1, wherein the controller is further configured to determine the focusing events based at least in part on a change in a parameter associated with the first set of the reflection signals.

4. The system of example 3, wherein the parameter associated with the first set of reflection signals comprises a phase or an amplitude.

5. The system of example 1, wherein the controller is further configured to:

computationally reconstruct an acoustic field at the at least one target region based at least in part on the first set of reflection signals; and
identify the focusing events based at least part on the reconstructed acoustic field.

6. The system of example 5, wherein the focusing events are identified only when the reconstructed acoustic field is converged at the at least one target region.

7. The system of example 1, wherein the controller is further configured to:

select the second set of reflection signals based at least in part on consistency therebetween, and
determine the parameter value associated with the at least one of the transducer elements based at least in part on the selected second set of reflection signals.

8. The system of example 1, wherein the controller is further configured to, prior to adjusting the parameter value associated with the at least one of the transducer elements, repeat (i) determining whether the target number or target occurrence rate of the focusing events has been reached and (ii) if not, causing the transducer to generate the second sonication at the adjusted acoustic power to the target region and measure the second set of reflection signals of the second sonication until the target number or target occurrence rate of the focusing events has been reached.

9. The system of example 8, wherein controller is further configured to adjust the parameter value associated with the at least one of the transducer elements based at least in part on the adjusted acoustic power.

10. The system of example 1, further comprising an administration device for introducing a transient acoustic reflector in proximity to or at the at least one target region, wherein at least one of the first or second set of reflection signals is from the transient acoustic reflector.

11. The system of example 10, wherein the administration device is an automatic administration device or a manual administration device.

12. The system of example 10, wherein the controller is further configured to:

determine whether artifacts are present in at least one of the first or second set of reflection signals; and

upon determining that the target number or target occurrence rate of the focusing events has not been reached and the artifacts are presented in at least one of the first or second sets of the reflection signals, cause the administration device to adjust a parameter associated with the transient acoustic reflector.

13. The system of example 12, wherein the controller is further configured to determine whether the artifacts are present in the first set of reflection signals prior to causing the transducer to generate the second sonication.

14. The system of example 12, wherein the parameter associated with the acoustic reflector comprises at least one of a concentration, a size or an agent type.

15. The system of example 14, wherein the controller is further configured to increase the concentration of the acoustic reflector upon determining that the target number or target occurrence rate of the focusing events has not been reached and the artifacts are presented in at least one of the first or second sets of reflection signals.

16. The system of example 10, wherein the controller is further configured to, prior to adjusting the parameter value associated with the at least one of the transducer elements:

determine whether the target number or target occurrence rate of the focusing events has been reached based on the second set of reflection signals;
determine whether the adjusted acoustic power associated with the second sonication is below a target power level; and
upon determining that the target number or target occurrence rate of the focusing events has been reached and the adjusted acoustic power associated with the second sonication is below the target power level, cause the administration device to decrease a concentration of the transient acoustic reflector.

17. The system of example 1, wherein the controller is further configured to cause the transducer to generate acoustic energy for creating a transient acoustic reflector in proximity to or at the at least one target region, wherein at least one of the first or second set of reflection signals is from the transient acoustic reflector.

18. The system of example 1, wherein the parameter value associated with the at least one transducer element comprises at least one of a phase or an amplitude of a signal driving the at least one transducer element.

19. The system of example 1, wherein the controller is further configured to cause at least some of the transducer elements to measure at least one of the first or second set of reflection signals.

20. The system of example 1, further comprising an acoustic-signal detector for measuring at least one of the first or second set of reflection signals.

21. The system of example 1, wherein the controller is further configured to:

(e) predict a therapeutic or acoustic change in the at least one target region resulting from adjustment of the parameter value;
(f) if the predicted change exceeds a maximum allowable change or causes the therapeutic or acoustic change in the at least one target region to exceed a target value, determine at least one of a power level, an ultrasound frequency or an energy level associated with the first one of the transducer elements and/or a second one of the transducer elements for reducing the predicted therapeutic or acoustic change; and
(g) activate the ultrasound transducer based at least in part on the parameter value determined in step (d) and the at least one of the power level, ultrasound frequency or energy level determined in step (f).

22. A method of focusing an ultrasound transducer comprising a plurality of transducer elements, the method comprising:

(a) generating a first sonication to at least one target region and measuring a first set of reflection signals resulting from the first sonication;
(b) based on the first set of reflection signals, determining whether a target number or a target occurrence rate of focusing events has been reached; and
(c) if not,

(i) generating a second sonication at an adjusted acoustic power to the at least one target region and measuring a second set of reflection signals resulting from the second sonication; and
(ii) based at least in part on the second set of reflection signals, adjusting a parameter value associated with at least one of the transducer elements so as to improve an ultrasound focus at the at least one target region.

23. The method of example 22, wherein the focusing events are reflection signals reflected by at least one transient acoustic reflector located in proximity to or at the at least one target region.

24. The method of example 22, further comprising determining the focusing events based at least in part on a change in a parameter associated with the first set of the reflection signals.

25. The method of example 24, wherein the parameter associated with the first set of reflection signals comprises a phase or an amplitude.

26. The method of example 22, further comprising:

computationally reconstructing an acoustic field at the at least one target region based at least in part on the first set of reflection signals; and
identifying the focusing events based at least part on the reconstructed acoustic field.

27. The method of example 26, wherein the focusing events are identified only when the reconstructed acoustic field is converged at the at least one target region.

28. The method of example 22, further comprising:

selecting the second set of reflection signals based at least in part on consistency therebetween, and determining the parameter value associated with the at least one of the transducer elements based at least in part on the selected second set of reflection signals.

29. The method of example 22, further comprising, prior to adjusting the parameter value associated with the at least one of the transducer elements, repeating (i) determining whether the target number or target occurrence rate of the focusing events has been reached and (ii) if not, causing the transducer to generate the second sonication at the adjusted acoustic power to the target region and measure the second set of reflection signals of the second sonication until the target number or target occurrence rate of the focusing events has been reached.

30. The method of example 29, further comprising adjusting the parameter value associated with the at least one of the transducer elements based at least in part on the adjusted acoustic power.

31. The method of example 22, further comprising introducing a transient acoustic reflector in proximity to or at the at least one target region, wherein at least one of the first or second set of reflection signals is from the transient acoustic reflector.

32. The method of example 31, further comprising:

determining whether artifacts are present in at least one of the first or second set of reflection signals; and upon determining that the target number or target occurrence rate of the focusing events has not been reached and the artifacts are presented in at least one of the first or second sets of the reflection signals, causing the administration device to adjust a parameter associated with the transient acoustic reflector.

33. The method of example 32, wherein whether the artifacts are present in the first set of reflection signals is determined prior to generating the second sonication.

34. The method of example 32, wherein the parameter associated with the acoustic reflector comprises at least one of a concentration, a size or an agent type.

35. The method of example 34, further comprising increasing the concentration of the acoustic reflector upon determining that the target number or target occurrence rate of the focusing events has not been reached and the artifacts are presented in at least one of the first or second sets of reflection signals.

36. The method of example 31, further comprising, prior to adjusting the parameter value associated with the at least one of the transducer elements:

determining whether the target number or target occurrence rate of the focusing events has been reached based on the second set of reflection signals; determining whether the adjusted acoustic power associated with the second sonication is below a target power level; and upon determining that the target number or target occurrence rate of the focusing events has been reached and the adjusted acoustic power associated with the second sonication is below the target power level, decreasing a concentration of the transient acoustic reflector.

37. The method of example 22, further comprising:

generating acoustic energy for creating a transient acoustic reflector in proximity to or at the at least one target region, wherein at least one of the first or second set of reflection signals is from the transient acoustic reflector.

38. The method of example 22, wherein the parameter value associated with the at least one transducer element comprises at least one of a phase or an amplitude of a signal driving the at least one transducer element.

39. The method of example 22, further comprising

(e) predicting a therapeutic or acoustic change in the at least one target region resulting from adjustment of the parameter value;

(f) if the predicted change exceeds a maximum allowable change or causes the therapeutic or acoustic change in the at least one target region to exceed a target value, determining at least one of a power level, an ultrasound frequency or an energy level associated with the first one of the transducer elements and/or a second one of the transducer elements for reducing the predicted therapeutic or acoustic change; and

(g) activating the ultrasound transducer based at least in part on the parameter value determined in step (d) and the at least one of the power level, ultrasound frequency or energy level determined in step (f).

40. A system for applying acoustic energy, the system comprising:

an ultrasound transducer comprising a plurality of transducer elements for providing a series of sonications to at least one target region; and

a controller configured to:

determine a phase value associated with a first one of the transducer elements to be adjusted for generating a focus at the at least one target region;

predict a therapeutic or acoustic change in the at least one target region resulting from adjustment of the phase value; and

if the predicted change exceeds a maximum allowable change or causes the therapeutic or acoustic change in the at least one target region to exceed a target value, (i) determine at least one of a power level, an ultrasound frequency or an energy level associated with the first one of the transducer elements and/or a second one of the transducer elements for reducing the predicted therapeutic or acoustic change, and (ii) activate the ultrasound transducer based at least in part on at least one of the determined phase value , power level, ultrasound frequency or energy level.

41. The system of example 40, wherein the controller is further configured to predict the therapeutic or acoustic change based at least in part on tissue aberrations resulting from intervening tissue located between the ultrasound transducer and the at least one target region.

42. The system of example 40, wherein the therapeutic or acoustic change is at least one of a temperature change, a pressure change, a mechanical index change, a cavitation activity change, a tissue sensitization change, a blood brain barrier disruption change, an acoustic radiation force change or a spot shape change.

43. The system of example 40, wherein the controller is further configured to establish a relationship between (i) the change in the at least one target region and (ii) the at least one of the power level, the ultrasound frequency or the energy level associated with the first one of the transducer elements and/or the second one of the transducer elements.

44. The system of example 43, wherein the relationship is established empirically or using a physical model.

45. The system of example 41, further comprising a transient acoustic reflector located in proximity to or at the at least one target region, the controller being further configured to:

cause the transducer to generate a first sonication in the sonication series to the at least one target region;

measure reflection signals of the first sonication from the transient acoustic reflector;

select the measured reflection signals based at least in part on consistency therebetween, and

based at least in part on the selected reflection signals, determine the phase value associated with the first one of the transducer elements.

46. The system of example 40, further comprising a transient acoustic reflector located in proximity to or at the at least one target region, the controller being further configured to:

cause the transducer to generate a first sonication in the sonication series to the at least one target region;

measure reflection signals of the first sonication from the transient acoustic reflector; computationally reconstruct an acoustic field at the at least one target region based at least in part on the measured reflection signals;

select the measured reflection signals based at least in part on the reconstructed acoustic field, and based at least in part on the selected reflection signals, determine the phase value associated with the first one of the transducer elements.

47. The system of example 40, further comprising a plurality of transient acoustic reflectors, each located in proximity to or at one of a plurality of the target regions, wherein the controller is further configured to:

sequentially generate a plurality of sonications to each of the transient acoustic reflectors and measure the reflection signals therefrom; and
select the reflection signals associated with the plurality of sonications from the plurality of transient acoustic reflectors based at least in part on consistency therebetween.

48. The system of example 47, wherein the controller is further configured to:

determine the phase value based at least in part on a first set of the selected reflection signals from a first one of the target regions; and
determine a second phase value associated with the first one of the transducer elements based at least in part on a second set of the selected reflection signals from a second one of the target regions.

49. The system of example 48, wherein the controller is further configured to predict a second therapeutic or acoustic change in the second one of the target regions resulting from adjustment of the second phase value based at least in part on the determined phase value, the second phase value and the predicted therapeutic or acoustic change.

50. The system of example 48, further comprising a measuring system for measuring the therapeutic or acoustic change in the first one of the target regions due to adjustment of the determined phase value.

51. The system of example 50, wherein the controller is further configured to predict a second therapeutic or acoustic change in the second one of the target regions resulting from adjustment of the second phase value based at least in part on the determined phase value, the second phase value and the measured therapeutic or acoustic change in the first one of the target regions.

52. The system of example 47, wherein the controller is further configured to:

computationally shift the location of a first one of the transient acoustic reflectors to coincide with the location of a second one of the transient acoustic reflectors;
computationally determine an updated phase value associated with the reflection signal from the shifted location of the first one of the transient acoustic reflectors; and
predict a therapeutic or acoustic change in the coincident location resulting from adjustments to the updated phase value and the determined phase value based at least in part on the determined phase value, the updated phase value and the predicted therapeutic or acoustic change.

53. The system of example 40, further comprising an imaging system for acquiring images of the at least one target region or a non-target region located between the transducer and the at least one target region.

54. The system of example 53, wherein the imaging system comprises at least one of a computer tomography (CT) device, a magnetic resonance imaging device (MRI), a positron emission tomography (PET) device, a single-photon emission computed tomography (SPECT) device, or an ultrasonography device.

55. The system of example 53, wherein the controller is further configured to determine, based at least in part on the acquired images, a spatial configuration of the at least one target region with respect to the transducer and tissue characteristics associated with the target region and the non-target region.

56. The system of example 55, wherein the controller is further configured to implement a physical model for (i) predicting the therapeutic or acoustic change in the target region and/or (ii) determining the at least one of the power level, the ultrasound frequency or the energy level based at least in part on the phase value, the spatial configuration of the target region with respect to the transducer and/or the tissue characteristics associated with the target region and the non-target region.

57. A method of applying acoustic energy from an ultrasound transducer comprising a plurality of transducer elements to at least one target region, the method comprising:

(a) determining a phase value associated with a first one of the transducer elements to be adjusted for generating an ultrasound focus at the at least one target region;

(b) predicting a therapeutic or acoustic change in the at least one target region resulting from adjustment of the phase value; and

(c) if the predicted therapeutic or acoustic change exceeds a maximum allowable change or causes the therapeutic or acoustic change in the at least one target region to exceed a target value, (i) determining at least one of a power level, an ultrasound frequency or an energy level associated with the first one of the transducer elements and/or a second one of the transducer elements for reducing the predicted therapeutic or acoustic change, and (ii) activating the ultrasound transducer based at least in part on at least one of the determined phase value, power level, ultrasound frequency, or energy level.

58. The method of example 57, wherein the at least one of the power level, the ultrasound frequency, the energy level determined in step (c) reduces the predicted therapeutic or acoustic change to a value not exceeding the target or maximum allowable therapeutic or acoustic change.

59. The method of example 57, wherein the therapeutic or acoustic change is at least one of a temperature change, a pressure change, a mechanical index change, a cavitation activity change, a tissue sensitization change, a blood brain barrier disruption change, an acoustic radiation force change or a spot shape change.

60. The method of example 57, wherein the therapeutic or acoustic change is predicted based at least in part on tissue aberrations resulting from intervening tissue located between the ultrasound transducer and the at least one target region.

61. The method of example 57, further comprising establishing a relationship between (i) the change in the at least one target region and (ii) the at least one of the power level, the ultrasound frequency or the energy level associated with the first one of the transducer elements and/or the second one of the transducer elements.

62. The method of example 61, wherein the relationship is established empirically or using a physical model.

63. The method of example 57, further comprising:

introducing a transient acoustic reflector in proximity to or at the at least one target region;
generating a first sonication to the at least one target region;
measuring reflection signals of the first sonication from the transient acoustic reflector;
selecting the measured reflection signals based at least in part on consistency therebetween, and
based at least in part on the selected reflection signals, determining the phase value associated with the first one of the transducer elements.

64. The method of example 57, further comprising:

introducing a transient acoustic reflector in proximity to or at the at least one target region;
generating a first sonication to the at least one target region;
measuring reflection signals of the first sonication from the transient acoustic reflector;
computationally reconstructing an acoustic field at the at least one target region based at least in part on the measured reflection signals;
selecting the measured reflection signals based at least in part on the reconstructed acoustic field, and
based at least in part on the selected reflection signals, determining the phase value associated with the first one of the transducer elements.

65. The method of example 57, further comprising:

sequentially generating a plurality of sonications to one or more transient acoustic reflectors located in proximity to or at one of a plurality of the target regions and measuring the reflection signals therefrom; and
selecting the reflection signals associated with the plurality of sonications from the transient acoustic reflectors based at least in part on consistency therebetween.

66. The method of example 65, further comprising:

determining the phase value in step (a) based at least in part on a first set of the selected reflection signals from a first one of the target regions; and

determining a second phase value associated with the first one of the transducer elements based at least in part on a second set of the selected reflection signals from a second one of the target regions.

67. The method of example 66, further comprising predicting a second therapeutic or acoustic change in the second one of the target regions resulting from adjustment of the second phase value based at least in part on the determined phase value, the second phase value and the predicted therapeutic or acoustic change.

68. The method of example 66, further comprising measuring the therapeutic or acoustic change in the first one of the target regions due to adjustment of the determined phase value.

69. The method of example 68, further comprising predict a second therapeutic or acoustic change in the second one of the target regions resulting from adjustment of the second phase value based at least in part on the determined phase value, the second phase value and the measured therapeutic or acoustic change in the first one of the target regions.

70. The method of example 65, further comprising:

computationally shifting the location of a first one of the transient acoustic reflectors to coincide with the location of a second one of the transient acoustic reflectors;
computationally determining an updated phase value associated with the reflection signal from the shifted location of the first one of the transient acoustic reflectors; and predicting a therapeutic or
acoustic change in the coincident location resulting from adjustments to the updated phase value and the determined phase value based at least in part on the determined phase value, the updated phase value and the predicted therapeutic or acoustic change.

71. The method of example 57, further comprising acquiring images of the at least one target region or a non-target region located between the transducer and the at least one target region.

72. The method of example 71, further comprising determining, based at least in part on the acquired images, a spatial configuration of the at least one target region with respect to the transducer and tissue characteristics associated with the target region and the non-target region.

73. The method of example 72, further comprising implementing a physical model for (i) predicting the therapeutic or acoustic change in the target region and/or (ii) determining the at least one of the power level, the ultrasound frequency or the energy level based at least in part on the phase value, the spatial configuration of the target region with respect to the transducer and/or the tissue characteristics associated with the target region and the non-target region.

74. A system for applying acoustic energy, the system comprising:

an ultrasound transducer comprising a plurality of transducer elements;
an imager; and
a controller configured to:

(a) populate a treatment profile specifying a plurality of treatment parameters associated with the ultrasound transducer and at least one target region;
(b) cause the imager to acquire images of a new target region and/or intervening tissue located between the transducer and the new target region;
(c) based at least in part on the acquired images, determine new treatment parameters associated with the new target region;
(d) identify best-matching treatment parameters in the treatment profile to the determined new treatment parameters;
(e) based at least in part on the identified best-matching treatment parameters, determine (i) adjustment of a phase value associated with at least one of the transducer elements for generating an optimal focus at the new target region and (ii) a therapeutic or acoustic change at the new target region resulting from adjustment of the phase value; and
(f) if the determined therapeutic or acoustic change exceeds a maximum allowable change or causes the therapeutic or acoustic change in the at least one target region to exceed a target value, determine adjustment of at least one of a power level, a frequency or an energy level associated with at least one of the transducer elements for reducing the therapeutic or acoustic change, and activate the ultrasound transducer based at least in part on the adjustment of the phase value determined in step (e) and the determined adjustment of the at least one of the power level, frequency, or energy level.

75. The system of example 74, wherein the treatment parameters comprise at least one of (i) geometry of the transducer elements and their locations and orientations relative to the at least one target region, (ii) tissue characteristics of the at least one target region and the intervening tissue, (iii) adjustments of the phase values associated with at least some of the transducer elements for generating an optimal focus at the at least one target region, (iv) therapeutic or acoustic changes at the at least one target region resulting from the phase value adjustments, or (v) adjustments of at least one of the acoustic powers, frequencies or energy levels associated with at least some of the transducer elements for reducing the therapeutic or acoustic changes.

76. The system of example 75, wherein the tissue characteristics comprise at least one of a location, a thickness, a density, or a material property.

77. The system of example 75, wherein the controller is further configured to empirically determine the adjustments of the phase values in step (iii), the therapeutic or acoustic changes at the at least one target region in step (iv), and the adjustments of the at least one of the acoustic powers, frequencies or energy levels in step (v).

78. The system of example 75, wherein the controller is further configured to implement a physical model for determining the adjustments of the phase values in step (iii), the therapeutic or acoustic changes at the at least one target region in step (iv), and the adjustments of the at least one of the acoustic powers, frequencies or energy levels in step (v) based at least in part on the geometry of the transducer elements and their locations and orientations relative to the at least one target region and the tissue characteristics of the at least one target region and the intervening tissue.

79. A method of applying acoustic energy utilizing an ultrasound transducer comprising a plurality of transducer elements, the method comprising:

(a) populating a treatment profile specifying a plurality of treatment parameters associated with the ultrasound transducer and at least one target region;
(b) acquiring images of a new target region and/or intervening tissue located between the transducer and the new target region;
(c) based at least in part on the acquired images, determining new treatment parameters associated with the new target region;
(d) identifying best-matching treatment parameters in the treatment profile to the determined new treatment parameters;
(e) based at least in part on the identified best-matching treatment parameters, determining (i) adjustment of a phase value associated with at least one of the transducer elements for generating an optimal focus at the new target region and (ii) a therapeutic or acoustic change at the new target region resulting from adjustment of the phase value; and
(f) if the determined therapeutic or acoustic change exceeds a maximum allowable change or causes the therapeutic or acoustic change in the at least one target region to exceed a target value, (i) determining adjustment of at least one of a power level, a frequency or an energy level associated with at least one of the transducer elements for reducing the therapeutic or acoustic change, and (ii) activating the ultrasound transducer based at least in part on the adjustment of the phase value determined in step (e) and the determined adjustment of the at least one of the power level, frequency, or energy level.

80. The method of example 79, wherein the treatment parameters comprise at least one of (i) geometry of the transducer elements and their locations and orientations relative to the at least one target region, (ii) tissue characteristics of the at least one target region and the intervening tissue, (iii) adjustments of the phase values associated with at least some of the transducer elements for generating an optimal focus at the at least one target region, (iv) therapeutic or acoustic changes at the at least one target region resulting from the phase value adjustments, or (v) adjustments of at least one of the acoustic powers, frequencies or energy levels associated with at least some of the transducer elements for reducing the therapeutic or acoustic changes.

81. The method of example 80, wherein the tissue characteristics comprise at least one of a location, a thickness, a density, or a material property.

82. The method of example 80, wherein the adjustments of the phase values in step (iii), the therapeutic or acoustic changes at the at least one target region in step (iv), and the adjustments of the at least one of the acoustic powers, frequencies or energy levels in step (v) are empirically determined.

83. The method of example 80, further comprising implementing a physical model to determine the adjustments of the phase values in step (iii), the therapeutic or acoustic changes at the at least one target region in step (iv), and the adjustments of the at least one of the acoustic powers, frequencies or energy levels in step (v) based at least in part on the geometry of the transducer elements and their locations and orientations relative to the at least one target region and the tissue characteristics of the at least one target region and the intervening tissue.

84. A system for focusing an ultrasound transducer, the system comprising:

an ultrasound transducer comprising a plurality of transducer elements for providing a series of sonications to at least one target region; and
a controller configured to:

(a) cause the transducer to generate a first sonication in the sonication series to the at least one target region and measure a first set of reflection signals resulting from the first sonication;
(b) based on the first set of reflection signals, determine whether a target number or a target occurrence rate of focusing events has been reached;
(c) if not, cause the transducer to generate a second sonication at an adjusted acoustic power to the at least one target region and measure a second set of reflection signals resulting from the second sonication;
(d) based at least in part on the second set of reflection signals, adjust a phase value associated with at least one of the transducer elements for improving an ultrasound focus at the at least one target region;
(e) predict a therapeutic or acoustic change in the at least one target region resulting from adjustment of the parameter value; and
(f) if the predicted change exceeds a maximum allowable change or causes the therapeutic or acoustic change in the at least one target region to exceed a target value, (i) determine at least one of a power level, an ultrasound frequency or an energy level associated with the first one of the transducer elements and/or a second one of the transducer elements for reducing the predicted therapeutic or acoustic change, and (ii) activate the ultrasound transducer based at least in part on the parameter value in step (d) and the at least one determined power level, ultrasound frequency or energy level.

85. A method of focusing an ultrasound transducer having a plurality of transducer elements, the method comprising:

(a) generating a first sonication to at least one target region and measuring a first set of reflection signals resulting from the first sonication;
(b) based on the first set of reflection signals, determining whether a target number or a target occurrence rate of focusing events has been reached;
(c) if not, generating a second sonication at an adjusted acoustic power to the at least one target region and measuring a second set of reflection signals resulting from the second sonication;
(d) based at least in part on the second set of reflection signals, adjusting a parameter value associated with at least one of the transducer elements so as to improve an ultrasound focus at the at least one target region;
(e) predicting a therapeutic or acoustic change in the at least one target region resulting from adjustment of the parameter value; and
(f) if the predicted change exceeds a maximum allowable change or causes the therapeutic or acoustic change in the at least one target region to exceed a target value, (i) determining at least one of a power level, an ultrasound frequency or an energy level associated with the first one of the transducer elements and/or a second one of the transducer elements for reducing the predicted therapeutic or acoustic change, and (ii) activating the ultrasound transducer based at least in part on the parameter value in step (d) and the determined power level, ultrasound frequency or energy level.

**Claims**

1. A system (100) for applying acoustic energy, the system (100) comprising:

an ultrasound transducer (102) comprising a plurality of transducer elements (104) for providing a series of sonications to at least one target region (101); and
a controller (108) configured to:

determine a phase value associated with a first one of the transducer elements (104) to be adjusted for generating a focus at the at least one target region (101);
predict a therapeutic or acoustic change in the at least one target region (101) resulting from adjustment of the phase value; and
if the predicted change exceeds a maximum allowable change or causes the therapeutic or acoustic change in the at least one target region (101) to exceed a target value, (i) determine at least one of a power level, an ultrasound frequency or an energy level associated with the first one of the transducer elements (104) and/or a second one of the transducer elements (104) for reducing the predicted therapeutic or acoustic change, and (ii) activate the ultrasound transducer (102) based at least in part on at least one of the determined phase value , power level, ultrasound frequency or energy level.

2. The system (100) of claim 1, wherein the controller (108) is further configured to predict the therapeutic or acoustic change based at least in part on tissue aberrations resulting from intervening tissue (210) located between the ultrasound transducer (102) and the at least one target region (101); and/or
wherein the therapeutic or acoustic change is at least one of a temperature change, a pressure change, a mechanical index change, a cavitation activity change, a tissue sensitization change, a blood brain barrier disruption change, an acoustic radiation force change or a spot shape change.

3. The system of claim 1, wherein the controller (108) is further configured to establish a relationship between (i) the change in the at least one target region (101) and (ii) the at least one of the power level, the ultrasound frequency or the energy level associated with the first one of the transducer elements (104) and/or the second one of the transducer elements (104).

4. The system of claim 3, wherein the relationship is established empirically or using a physical model.

5. The system (100) of claim 1 or 2, further comprising a transient acoustic reflector (202) located in proximity to or at the at least one target region (101), the controller being further configured to:

cause the transducer (102) to generate a first sonication in the sonication series to the at least one target region (101);
measure reflection signals of the first sonication from the transient acoustic reflector (202);
select the measured reflection signals based at least in part on consistency therebetween, and
based at least in part on the selected reflection signals, determine the phase value associated with the first one of the transducer elements (104).

6. The system (100) of claim 1, further comprising a transient acoustic reflector (202) located in proximity to or at the at least one target region (101), the controller being further configured to:

cause the transducer (102) to generate a first sonication in the sonication series to the at least one target region (101);
measure reflection signals of the first sonication from the transient acoustic reflector (202);
computationally reconstruct an acoustic field at the at least one target region $< H_d )$ based at least in part on the measured reflection signals;
select the measured reflection signals based at least in part on the reconstructed acoustic field, and
based at least in part on the selected reflection signals, determine the phase value associated with the first one of the transducer elements (104).

7. The system (100) of claim 1, further comprising a plurality of transient acoustic reflectors (202), each located in proximity to or at one of a plurality of the target regions (101), wherein the controller (108) is further configured to:

sequentially generate a plurality of sonications to each of the transient acoustic reflectors (202) and measure the reflection signals therefrom; and

select the reflection signals associated with the plurality of sonications from the plurality of transient acoustic reflectors (202) based at least in part on consistency therebetween.

8. The system of claim 7, wherein the controller (108) is further configured to:

determine the phase value based at least in part on a first set of the selected reflection signals from a first one of the target regions (101); and

determine a second phase value associated with the first one of the transducer elements (104) based at least in part on a second set of the selected reflection signals from a second one of the target regions (101).

9. The system of claim 8, wherein the controller (108) is further configured to predict a second therapeutic or acoustic change in the second one of the target regions (101) resulting from adjustment of the second phase value based at least in part on the determined phase value, the second phase value and the predicted therapeutic or acoustic change.

10. The system of claim 8, further comprising a measuring system for measuring the therapeutic or acoustic change in the first one of the target regions (101) due to adjustment of the determined phase value.

11. The system of claim 10, wherein the controller (108) is further configured to predict a second therapeutic or acoustic change in the second one of the target regions (101) resulting from adjustment of the second phase value based at least in part on the determined phase value, the second phase value and the measured therapeutic or acoustic change in the first one of the target regions (101).

12. The system of claim 8, wherein the controller (108) is further configured to:

computationally shift the location of a first one of the transient acoustic reflectors (202) to coincide with the location of a second one of the transient acoustic reflectors (202);

computationally determine an updated phase value associated with the reflection signal from the shifted location of the first one of the transient acoustic reflectors (202); and

predict a therapeutic or acoustic change in the coincident location resulting from adjustments to the updated phase value and the determined phase value based at least in part on the determined phase value, the updated phase value and the predicted therapeutic or acoustic change.

13. The system (100) of claim 1, further comprising an imaging system for acquiring images of the at least one target region (101) or a non-target region located between the transducer (102) and the at least one target region (101).

14. The system of claim 13, wherein the imaging system comprises at least one of a computer tomography (CT) device, a magnetic resonance imaging device (MRI), a positron emission tomography (PET) device, a single-photon emission computed tomography (SPECT) device, or an ultrasonography device.

15. The system of claim 13, wherein the controller (108) is further configured to determine, based at least in part on the acquired images, a spatial configuration of the at least one target region (101) with respect to the transducer and tissue characteristics associated with the target region (101) and the non-target region; and/or

wherein the controller (108) is further configured to implement a physical model for (i) predicting the therapeutic or acoustic change in the target region (101) and/or (ii) determining the at least one of the power level, the ultrasound frequency or the energy level based at least in part on the phase value, the spatial configuration of the target region (101) with respect to the transducer and/or the tissue characteristics associated with the target region (101) and the non-target region.

FIG. 1

EP 4 649 997 A2

FIG. 2A

FIG. 2B

FIG. 3A

EP 4 649 997 A2

FIG. 3B

3D RECONSTRUCTION

FIG. 4B

3D RECONSTRUCTION

FIG. 4A

FIG. 5

~ 600

GENERATE/INTRODUCE TRANSIENT REFLECTOR(s) HAVING A CONCENTRATION IN PROXIMITY ONE OR MORE SONICATION LOCATIONS THAT ARE IN PROXIMITY TO OR AT THE TARGET REGION — 602

TRANSMIT A SERIES OF LOW-POWER SONICATIONS TO THE TRANSIENT REFLECTOR(s) AND MEASURE THE REFLECTION SIGNALS THEREFROM — 604

SELECT SIGNALS FROM THE ACOUSTIC REFLECTOR(s) BASED ON THE DIFFERENCE BETWEEN THE AMPLITUDES AND/OR PHASES ASSOCIATED WITH THE REFLECTION SIGNALS IN TWO CONSECUTIVE MEASUREMENTS — 606

SELECT REFLECTION SIGNALS FROM SINGLE MICROBUBBLES BASED ON THE CONSISTENCY BETWEEN THE REFLECTION SIGNALS AND/OR THE RECONSTRUCTED ACOUSTIC FIELD(s) AT THE SONICATION REGION(s) — 608

DETERMINE WHETHER A SUFFICIENT NUMBER OR A SUFFICIENT OCCURRING RATE OF THE FOCUSING EVENTS IS MEASURED — 610

612    NO ↓                                                      ↓ YES    618

DETERMINE WHETHER A THRESHOLD POWER CORRESPONDING TO THE NONLINEAR RESPONSES OF THE ACOUSTIC REFLECTORS HAS BEEN REACHED

DETERMINE WHETHER TOO MANY FOCUSING EVENTS HAVE OCCURRED WHEN THE ACOUSTIC POWER IS STILL LOW

NO ↓                ↓ YES              YES ↓                ↓ NO

INCREASE THE ACOUSTIC POWER OF THE APPLIED SONICATIONS

INCREASE THE CONCENTRATION OF THE ACOUSTIC REFLECTORS

REDUCE THE CONCENTRATION OF THE ACOUSTIC REFLECTORS

INCREASE THE ACOUSTIC POWER OF THE APPLIED SONICATIONS UNTIL THE OPTIMAL POWER $P_F$ IS REACHED

616 ⌐          614 ⌐          620 ⌐          622 ⌐

COMMENCE THE AUTOFOCUSING PROCEDURE BASED ON THE DETERMINED OPTIMAL ACOUSTIC POWER AND OPTIMAL CONCENTRATION OF THE ACOUSTIC REFLECTORS — 624

ADJUST ULTRASOUND PARAMETERS BASED ON THE FOCUSING EVENTS MEASURED DURING THE AUTOFOCUSING PROCEDURE — 626

FIG. 6

FIG. 7A

FIG. 7B

FIG. 8

900

ESTABLISH AND STORE, IN A DATABASE, A RELATIONSHIP BETWEEN ONE OR MORE ULTRASOUND PARAMETERS AND THE RESULTING TEMPERATURE CHANGES AT THE TARGET ~902

GENERATE/INTRODUCE TRANSIENT ACOUSTIC REFLECTORS HAVING THE OPTIMAL CONCENTRATION INTO THE SONICATION LOCATIONS IN PROXIMATE TO OR AT THE TARGET REGION AND ACTIVATE THE TRANSDUCER ELEMENTS WITH THE OPTIMAL PARAMETER(s) ~904

MEASURE AND SELECT REFLECTION SIGNALS FROM THE ACOUSTIC REFLECTORS ~906

ANALYZE THE SELECTED REFLECTION SIGNALS TO DETERMINE THE ULTRASOUND PARAMETERS ~908

PREDICT AND/OR MEASURE THE PEAK POWER/TEMPERATURE CHANGE IN THE FIRST SONICATION LOCATION ~910

ESTIMATE THE PEAK POWER/TEMPERATURE CHANGES IN THE OTHER SONICATION LOCATIONS BASED AT LEAST IN PART ON THE MEASURED PEAK POWER/TEMPERATURE CHANGE IN THE FIRST SONICATION LOCATION ~912

DETERMINES WHETHER THE PREDICTED/MEASURED CHANGE IN THE PEAK POWER/TEMPERATURE AT EACH SONICATION LOCATION EXCEEDS A TARGET OR A MAXIMUM ALLOWABLE CHANGE ~914

ADJUST THE ULTRASOUND PARAMETER VALUE(s) SO AS TO REDUCE THE PREDICTED CHANGE AT THE TARGET ~916

OPERATE THE TRANSDUCER ELEMENT(s) BASED ON THE ADJUSTED PARAMETER VALUE(s) ~918

FIG. 9A

930

ESTABLISH AND STORE, IN A DATABASE, A RELATIONSHIP BETWEEN ONE OR MORE ULTRASOUND PARAMETERS AND THE RESULTING TEMPERATURE CHANGES AT THE TARGET ~902

GENERATE/INTRODUCE TRANSIENT ACOUSTIC REFLECTORS HAVING THE OPTIMAL CONCENTRATION INTO THE SONICATION LOCATIONS IN PROXIMATE TO OR AT THE TARGET REGION AND ACTIVATE THE TRANSDUCER ELEMENTS WITH THE OPTIMAL PARAMETER(s) ~904

MEASURE AND SELECT REFLECTION SIGNALS FROM THE ACOUSTIC REFLECTORS ~906

COMPUTATIONALLY SHIFT ALL (OR AT LEAST SOME) TRANSIENT ACOUSTIC REFLECTORS TO COINCIDE AT A SINGLE LOCATION ~932

COMPUTATIONALLY DETERMINE THE PHASES ASSOCIATED WITH THE SHIFTED REFLECTION SIGNALS AND/OR THE UNSHIFTED REFLECTION SIGNAL AT THE COINCIDENT LOCATION ~934

DETERMINE THE PHASE ASSOCIATED WITH EACH TRANSDUCER ELEMENT BASED ON THE PHASES ASSOCIATED WITH THE SHIFTED REFLECTION SIGNALS AND/OR THE UNSHIFTED REFLECTION SIGNAL DETERMINED IN STEP 934 ~936

DETERMINE AN ACOUSTIC POWER/TEMPERATURE INCREASE RESULTING FROM EACH OF THE SHIFTED AND/OR UNSHIFTED REFLECTION SIGNALS ~938

DETERMINE THE ACOUSTIC POWER/TEMPERATURE INCREASE AT THE COINCIDENT LOCATION BASED ON THE ACOUSTIC POWER/ TEMPERATURE INCREASES RESULTING FROM THE SHIFTED AND/OR UNSHIFTED REFLECTION SIGNALS DETERMINED IN STEP 938 ~940

DETERMINES WHETHER THE PREDICTED/MEASURED CHANGE IN THE PEAK POWER/TEMPERATURE AT THE COINCIDENT LOCATION EXCEEDS A TARGET OR A MAXIMUM ALLOWABLE CHANGE ~942

ADJUST THE ULTRASOUND PARAMETER VALUE(s) SO AS TO REDUCE THE PREDICTED CHANGE AT THE TARGET ~916

OPERATE THE TRANSDUCER ELEMENT(s) BASED ON THE ADJUSTED PARAMETER VALUE(s) ~918

FIG. 9B

950

ESTABLISH AND STORE, IN A DATABASE, A TREATMENT PROFILE
OF PATIENTS WHO PREVIOUSLY EXPERIENCED
THE ULTRASOUND PROCEDURE — 952

ACQUIRE IMAGES ASSOCIATED WITH THE TARGET OF
THE CURRENT PATIENT — 954

DETERMINE ONE OR MORE TREATMENT PARAMETERS
ASSOCIATED WITH THE TARGET OF THE CURRENT PATIENT
BASED ON THE ACQUIRED IMAGES — 956

IDENTIFY THE TREATMENT PARAMETERS THAT MOST CLOSELY
MATCH THE TREATMENT PARAMETERS OF THE CURRENT PATIENT — 958

DETERMINE THE REQUIRED PHASE ADJUSTMENTS FOR GENERATING
AN OPTIMAL FOCUS AT THE TARGET AS WELL AS THE REQUIRED
ADJUSTMENTS OF THE POWER LEVEL/FREQUENCY/ENERGY
LEVELS ADJUSTMENTS ASSOCIATED WITH THE TRANSDUCER
ELEMENTS FOR COMPENSATING THE PEAK POWER/TEMPERATURE
INCREASE RESULTING FROM IMPROVEMENT OF THE FOCUSING
PROPERTIES AT THE FOCUS — 960

OPERATE THE TRANSDUCER ELEMENT(s) BASED ON THE BASED
ON THE DETERMINED ADJUSTMENTS OF THE PHASES, POWER
LEVELS, FREQUENCIES, AND/OR ENERGY LEVELS — 962

FIG. 9C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62949593
- US 62949595 B
- US 20190308038 A
- WO 2020128615 A
- WO 2019116095 A
- US 62949597
- WO 2019234497 A
- WO 2018020315 A
- US 20120029396 A